# EUROPEAN PATENT APPLICATION

(11) **EP 4 652 873 A1**
(43) Date of publication of application: **26.11.2025**
(21) Application number: 24177548.5
(22) Date of filing: 23.05.2024
(51) Int. Cl.: A24F 40/30, A24F 40/50, A24F 40/10, A24F 40/05, A61M 15/00

(54) **AEROSOL GENERATING APPARATUS**

(71) Applicant: Imperial Tobacco Limited, Bristol, BS3 2LL (GB)
(72) Inventor: The designation of the inventor has not yet been filed
(74) Representative: Haseltine Lake Kempner LLP

(57) **Abstract**

The invention provides an aerosol generating system comprising: a first aerosol generator for generating a first aerosol from a first formulation wherein the first formulation comprises a nicotine formulation, the first aerosol being for inhalation by a user; a second aerosol generator for generating a second aerosol from a second formulation, different from the first formulation wherein the second formulation comprises a flavour formulation, the second aerosol being for inhalation by a user in combination with the first aerosol; and electrical circuitry, wherein the electrical circuitry is adapted to: delay the activation of the first aerosol generator with respect to the activation of the second aerosol generator for a first delay period in response to an inhalation of a user of the aerosol generating system

## Description

### FIELD

The present disclosure relates to an aerosol generating apparatus.

### BACKGROUND

A typical aerosol generating apparatus may comprise a power supply, an aerosol generating unit that is driven by the power supply, an aerosol precursor, which in use is aerosolised by the aerosol generating unit to generate an aerosol, and a delivery system for delivery of the aerosol to a user.

In some cases, the aerosol generating unit may include an ultrasonic generator e.g. a piezoelectric transducer (PET) for generating the aerosol. In use, the surface of the PET will expand and contract as it vibrates.

A PET generates aerosol by causing cavitation to occur within a liquid aerosol precursor that is provided on a surface of the PET. Cavitation refers to the phenomenon where the static pressure of a liquid reduces to below the liquid's vapour pressure, leading to the formation of small vapour filled cavities within the liquid. When the cavities are subsequently subjected to a higher pressure, the cavities collapse resulting in a shock wave that propagates through the liquid. This shock wave induces capillary waves, or ripples, in a surface distal (referred to herein as the upper surface of the liquid) from the PET that may form ligaments to expel droplets from the upper surface. More succinctly, in a thin layer of liquid, the collapsing of the cavities can induce a disturbance in the liquid that causes liquid droplets to be expelled from liquid, thereby forming an aerosol over the surface of the liquid, typically within an aerosolisation chamber.

In the context of a PET for generating the aerosol, when the surface of the PET expands, the liquid on the surface will conform to the expanded surface. When the surface of the PET subsequently contracts, the static pressure in the liquid will fall as it is effectively dragged with the surface with the decrease in static pressure being proportional to the speed of the movement of the PET surface (i.e., the frequency of the vibration). If the frequency and the amplitude of vibration of the PET are sufficiently high, cavitation will occur as a result of the contraction.

When the surface of the PET subsequently expands, the static pressure in the liquid will rise as it is effectively compressed by the surface with the increase in static pressure being proportional to the speed of the movement of the PET surface (i.e., the frequency of the vibration). Any cavities in the liquid previously formed may then implode, generating shock waves in the liquid capable of expelling droplets to form an aerosol.

In an aerosol generating apparatus using a PET, a liquid aerosol precursor is typically applied to the PET surface using a wick in fluid communication with a tank. The aerosol generated by cavitation of the liquid aerosol precursor will be drawn from the aerosolisation chamber along an aerosol flow path by suction at a mouthpiece outlet.

Aerosol generating apparatuses that use a PET for generating the aerosol present numerous challenges, including accurately driving the vibrational element and inefficiencies in the requisite circuitry.

In spite of the effort already invested in the development of aerosol generating apparatuses/systems further improvements are desirable.

### SUMMARY

In a first aspect the present disclosure provides aerosol generating system that comprises a first aerosol generator for generating a first aerosol from a first formulation and a second aerosol generator for generating a second aerosol from a second formulation, different from the first formulation.

In some examples, the aerosol generating system further comprises electrical circuitry adapted to alternate the activation of only the first aerosol generator and both the first aerosol generator and the second aerosol generator over a series of inhalations of a user of the aerosol generating system.

In other words, the aerosol generating system may be adapted to alternate between generating the first aerosol alone and generating a combination of the first and second aerosols.

Put another way, the aerosol generating system may be adapted to alternate between generating a pure aerosol, i.e., an aerosol comprised of aerosol generated from a single aerosol precursor liquid, such as the first aerosol precursor liquid, and a mixed aerosol, i.e., an aerosol comprises of aerosols generated from multiple different aerosol precursor liquids, such as the first and second aerosol precursor liquids.

In this way, the device may alternate between providing different aerosol compositions to the user over a series of multiple inhalations. By alternating between providing only the first aerosol and both the first and the second aerosol, the user's perception of the aerosol being inhaled, and in particular, the user perception of the second aerosol may be improved.

More specifically, the user perception of the second aerosol may be improved by creating a change in the composition of the aerosol being inhaled between inhalations. For example, if a user takes several inhalations of aerosol containing both the first and the second aerosol, the user may become used to the presence of the second aerosol, and their perception of the second aerosol may decrease. However, by providing an inhalation comprising only the first aerosol, and absent the second aerosol, the subsequent inhalation comprising both the first and the second aerosol will provide the user with a perceived increase in the amount of second aerosol present in the mixture, even if the amount of second aerosol generated either side of the inhalation comprising only the first aerosol remains unchanged.

When referring to perceived changes in the aerosol by the user, it is to be understood that each inhalation comprising both the first aerosol and the second aerosol may be substantially identical, i.e., may comprise the same mixture, or composition, of first and second aerosols. However, when provided in the context of immediately following an inhalation comprising only the first aerosol, the presence of the second aerosol will be more noticeable to the user. Similarly, when provided in the context of an uninterrupted series of inhalations comprising both the first and second aerosols, the presence of the second aerosol will be less noticeable to the user. Therefore, by providing an occasional absence of the second aerosol, the user's perception of the second aerosol will be renewed or refreshed.

In some examples, the first formulation comprises a nicotine formulation. In some examples, the second formulation comprises a flavour formulation. In this way, the device may be adapted to alternate between generating an aerosol containing only nicotine formulation and an aerosol containing a mixture of nicotine formulation and flavour formulation.

As discussed above, in this way the device may be adapted to improve the user perception of the flavour of the second aerosol in the mixed aerosol by providing a change in perceived flavour intensity over a series of inhalations. In this way, the user perception of the flavour may be kept fresh over a longer period of time.

In other words, by providing the user with a change, or modulation, in the flavour profile of the aerosol being inhaled, the user's sensitivity to the flavour may be maintained over a longer period of time. Put another way, modulating the flavour profile of the aerosol being inhaled, by occasionally selectively preventing flavour from being included in an inhalation, the time taken for a user to get used to a flavour may be increased.

In some examples, alternating the activation of only the first aerosol generator and both the first aerosol generator and the second aerosol generator comprises switching from activating both the first aerosol generator and the second aerosol generator to activating only the first aerosol generator and on non-consecutive inhalations of the user of the aerosol generating system.

In this way, the user may be allowed to become used to receiving the mixture of the first and second aerosols over a series of inhalations before the device alternates between generating only the first aerosol and generating both the first and the second aerosol. Thus, the user may be provided with a greater perceived change, or refresh, in the intensity of the second aerosol, e.g., the flavour.

In some examples, alternating the activation of only the first aerosol generator and both the first aerosol generator and the second aerosol generator comprises: activating both the first aerosol generator and the second aerosol generator for one or more inhalations of the user of the aerosol generating system before switching to activating only the first aerosol generator; and activating only the first aerosol generator for a single inhalation of the user of the aerosol generating system before switching to activating both the first aerosol generator and the second aerosol generator.

In this way, the device may be limited to generating an aerosol with only the first aerosol liquid precursor for only a single inhalation before switching to generating an aerosol with both the first and second aerosol liquid precursor. In this way, the number of inhalations without the second aerosol present may be minimized whilst still providing the user with a perceived change, or refresh, in intensity of the second aerosol, e.g., the flavour.

In some examples, alternating the activation of only the first aerosol generator and both the first aerosol generator and the second aerosol generator comprises switching between activating only the first aerosol generator and activating both the first aerosol generator and the second aerosol generator on consecutive inhalations of the user of the aerosol generating system.

In this way, the device may alternate between generating only the first aerosol and generating both the first and the second aerosol with each inhalation of the user. Thus, the user may be provided with a perceived change, or refresh, in intensity of the second aerosol, e.g., the flavour, with every other inhalation.

In some examples, the electrical circuitry is further adapted to: determine an elapsed time since an inhalation last occurred; and switch to activating both the first aerosol generator and the second aerosol generator for a next inhalation of the user of the aerosol generating system based on the elapsed time.

In this way, the device may be adapted to reset the alternation between activating only the first aerosol generator and activating both the first aerosol generator and the second aerosol generator after a given period of inactivity, i.e., the elapsed time since an inhalation last occurred.

The user's perception of the second aerosol will be naturally refreshed after a period of time following an inhalation. Thus, by switching to activating both the first aerosol generator and the second aerosol generator after a given period of inactivity, the user will be provided with a perceived change in intensity of the second aerosol, e.g., the flavour, naturally. Further, in this way, the device may be prevented from generating only the first aerosol in response to the first inhalation of a user in an inhalation session, thereby preventing the first inhalation of an inhalation session from lacking the second aerosol, which may for example be the flavour.

In some examples, switching to activating both the first aerosol generator and the second aerosol generator based on the elapsed time comprises: determining whether the elapsed time exceeds a predetermined period; if the elapsed time exceeds the predetermined period, switching to activating both the first aerosol generator and the second aerosol generator; and if the elapsed time exceeds the predetermined period, maintaining a current activation state of the first and second aerosol generators.

In this way, the device may recognise a new inhalation session, and respond by activating both the first aerosol generator and the second aerosol generator as the user perception of the second aerosol will already be naturally refreshed by virtue of the elapsed time since an inhalation last occurred being sufficiently long.

In some examples, the aerosol generating system further comprises a pressure sensor adapted to sense an inhalation of the user and send an inhalation signal to the electrical circuitry in response to sensing the inhalation of the user, and wherein the electrical circuitry is adapted to activate only the first aerosol generator, or both the first aerosol generator and the second aerosol generator, in response to the inhalation signal. In this way, the device may respond automatically to an inhalation of the user based on the pressure sensor, rather than requiring an additional user input.

In some examples, the aerosol generating system further comprises a communication unit adapted to receive a control signal, and wherein the electrical circuitry is adapted to adjust a function of the aerosol generating system in response to the control signal. In this way, the device may adapt in response to a control signal, for example received from a connected user device, for example generated by way of an application on a user's smartphone.

In some examples, adjusting a function of the aerosol generating system comprises adjusting a number of inhalations for which both the first aerosol generator and the second aerosol generator are activated before switching to activating only the first aerosol generator. In this way, the device may be adapted to alternate between activating only the first aerosol generator or activating both the first aerosol generator and the second aerosol generator according to a user preference.

In some examples, adjusting a function of the aerosol generating system comprises adjusting the predetermined period. In this way, the device may be adapted to alternate between activating only the first aerosol generator or activating both the first aerosol generator and the second aerosol generator according to a user preference.

In some examples, the first aerosol generator comprises a first piezoelectric transducer and the second aerosol generator comprises a second piezoelectric transducer. In this way, the device may generate the first and second aerosols by way of ultrasonic cavitation. By using piezoelectric transducers as the first and second aerosol generators, the speed at, or reactivity with, which the first and second aerosol generator can be activated and deactivated may be improved, for example when compared to heaters which have lag times associated with heating up and cooling down.

The present disclosure may provide a method for controlling an aerosol generating system, the aerosol generating system comprising: a first aerosol generator for generating a first aerosol from a first formulation; and a second aerosol generator for generating a second aerosol from a second formulation, different from the first formulation, which may implement any one or more features disclosed herein. The method may comprise alternating the activation of only the first aerosol generator and both the first aerosol generator and the second aerosol generator over a series of inhalations of a user of the aerosol generating system. The method may further comprise activating both the first aerosol generator and the second aerosol generator for one or more inhalations of the user of the aerosol generating system before switching to activating only the first aerosol generator; and activating only the first aerosol generator for a single inhalation of the user of the aerosol generating system before switching to activating both the first aerosol generator and the second aerosol generator.

The present disclosure may provide electrical circuitry and/or a computer program configured to cause an aerosol generating apparatus/system to perform any method or method step disclosed herein. A computer readable medium comprising the computer program is also disclosed.

In a second aspect the present disclosure provides aerosol generating system that comprises a first aerosol generator for generating a first aerosol from a first formulation, wherein the first formulation comprises a nicotine formulation, the first aerosol being for inhalation by a user and a second aerosol generator for generating a second aerosol from a second formulation, different from the first formulation, wherein the second formulation comprises a flavour formulation, the second aerosol being for inhalation by a user in combination with the first aerosol. The aerosol generating system further comprises electrical circuitry adapted to delay the activation of the first aerosol generator with respect to the activation of the second aerosol generator for a first delay period in response to an inhalation of a user of the aerosol generating system.

In other words, the aerosol generating system may be adapted to produce the second aerosol for a short period before the first aerosol is produced, during a given inhalation.

Put another way, the aerosol generating system may be adapted to deliver the second aerosol ahead of the first aerosol.

In this way, the device may improve the user perception of the aerosol inhaled by masking one aerosol with another aerosol. In particular, the first aerosol, for example the taste of the first aerosol, may be masked, blocked or covered using the second aerosol by starting to provide the second aerosol to the user before the first aerosol. After the delay period, the first and second aerosols may be delivered to the user simultaneously for the remainder of the inhalation.

In some examples, the first formulation comprises a nicotine formulation. In some examples, the second formulation comprises a flavour formulation. In this way, the device may be adapted to delay generating the first aerosol containing only nicotine formulation for a first delay period, during which the device is generating the second aerosol, which contains the flavour formulation.

Put another way, the device may be adapted to provide the user with a flavoured aerosol ahead of an aerosol containing only nicotine formulation. As discussed above, in this way the device may be adapted to mask the taste of the nicotine formulation by delaying the generation of the first aerosol with respect to the generation of the second aerosol. In this way, the user perception of the combined aerosol, comprising the first and second aerosols, may be improved.

In some examples, the electrical circuitry is further adapted to: measure an inhalation duration of the user over a series of inhalations; determine an optimal first delay period for delaying the activation of the first aerosol generator with respect to the activation of the second aerosol generator based on an average inhalation duration of the user; and delaying the activation of the first aerosol generator with respect to the activation of the second aerosol generator for the optimal first delay period.

In this way, the device may adapt to the user's typical inhalation period over time in order to determine an optimal first delay period such that the first aerosol is masked by the second aerosol, but a sufficient amount of first aerosol is also delivered within the user's typical inhalation period. For example, for a user with a shorter average inhalation period, the first delay period may be reduced, and for a user with a longer average inhalation period, the first delay period may be extended.

In some examples, the electrical circuitry is further adapted to alternate the delaying of the activation of the first aerosol generator with respect to the activation of the second aerosol generator for the first delay period with delaying the activation of the second aerosol generator with respect to the activation of the first aerosol generator for a second delay period. In this way, the device may selectively delay the generation of either the first aerosol with respect to the second aerosol or the second aerosol with respect to the first aerosol according to the implementation of the device. By providing the first aerosol ahead of the second aerosol, the user may be provided with a perceived change in the intensity of the second aerosol, thereby improving the user's perception of the second aerosol.

More specifically, the user perception of the second aerosol may be improved by creating a change in the composition of the aerosol being inhaled between inhalations. For example, if a user takes several inhalations of aerosol with the second aerosol generated before the first aerosol, the user may become used to the presence of the second aerosol, and their perception of the second aerosol may decrease. However, by providing an inhalation where the first aerosol is generated ahead of the second aerosol, the subsequent inhalation where the second aerosol is provided before the first aerosol will provide the user with a perceived increase in the amount of second aerosol present in the mixture.

When referring to perceived changes in the aerosol by the user, it is to be understood that each inhalation may be substantially identical, i.e., may comprise the same mixture, or composition, of first and second aerosols. However, when provided in the context of immediately following an inhalation where the first aerosol is generated ahead of the second aerosol, in an inhalation where the second aerosol is generated ahead of the first aerosol the presence of the second aerosol will be more noticeable to the user. Similarly, when provided in the context of an uninterrupted series of inhalations where the second aerosol is generated before the first aerosol, the presence of the second aerosol may become less noticeable to the user. Therefore, by providing an occasional inhalation where the first aerosol is generated before the second aerosol, i.e., the generation of the second aerosol is delayed with respect to the first aerosol, the user's perception of the second aerosol will be renewed or refreshed.

In some examples, alternating between the delaying of the activation of the first aerosol generator with respect to the activation of the second aerosol generator and delaying the activation of the second aerosol generator with respect to the activation of the first aerosol generator is performed on non-consecutive inhalations of the user of the aerosol generating system. In this way, the user may be allowed to become used to receiving the second aerosol ahead of the first aerosol before the device switches to delaying the second aerosol with respect to the first aerosol. Thus, the user may be provided with a greater perceived change, or refresh, in intensity of the second aerosol, e.g., the flavour.

In some examples, alternating between the delaying of the activation of the first aerosol generator with respect to the activation of the second aerosol generator and delaying the activation of the second aerosol generator with respect to the activation of the first aerosol generator comprises: delaying the activation of the first aerosol generator with respect to the activation of the second aerosol generator for one or more inhalations of the user of the aerosol generating system before switching to delaying the activation of the second aerosol generator with respect to the activation of the first aerosol generator; and delaying the activation of the second aerosol generator with respect to the activation of the first aerosol generator for a single inhalation of the user of the aerosol generating system before switching to delaying the activation of the first aerosol generator with respect to the activation of the second aerosol generator.

In this way, the device may be limited to generating an aerosol with the first aerosol liquid precursor delivered ahead of the second aerosol liquid precursor for only a single inhalation at a time. In this way, the number of inhalations without the second aerosol masking the first aerosol may be minimized whilst still providing the user with a perceived change, or refresh, in intensity of the second aerosol, e.g., the flavour.

In some examples, the electrical circuitry is further adapted to: determine an elapsed time since an inhalation last occurred; and if the elapsed time exceeds a predetermined period, switching to delaying the activation of the first aerosol generator with respect to the activation of the second aerosol generator; and if the elapsed time exceeds the predetermined period, maintaining a current delayed activation state of the first and second aerosol generators.

In this way, the device may be adapted to reset the delayed activation state of the first and second aerosol generators after a given period of inactivity, i.e., the elapsed time since an inhalation last occurred.

The user's perception of the second aerosol will be naturally refreshed after a period of time following an inhalation. Thus, by switching to delaying the activation of the first aerosol generator with respect to the second aerosol generator after a given period of inactivity, the user will be provided with a perceived change in intensity of the second aerosol, e.g., the flavour, naturally. Further, in this way, the device may be prevented from generating the first aerosol ahead of the second aerosol in response to the first inhalation of a user in an inhalation session, thereby preventing the first inhalation of a session from lacking the second aerosol, which may for example be the flavour, to mask the first aerosol, which may for example comprise the nicotine.

In some examples, the aerosol generating system further comprises a pressure sensor adapted to sense an inhalation of the user and send an inhalation signal to the electrical circuitry in response to sensing the inhalation of the user, and wherein the electrical circuitry is adapted to activate the first and second aerosol generators in response to the inhalation signal. In this way, the device may respond automatically to an inhalation of the user based on the pressure sensor, rather than requiring an additional user input.

In some examples, the electrical circuitry is further adapted to: determine an inhalation strength based on the inhalation signal; and adjust the first delay period based on the inhalation strength. In this way, the device may adapt the timing of the provision of the first and second aerosols according to the inhalation strength of the user. For example, for a strong inhalation, i.e., an inhalation with a strength above a predetermined threshold, the first delay period may be increased in order to prevent over delivery of the first aerosol. In another example, for a weak inhalation, i.e., an inhalation with a strength below a predetermined threshold, the first delay period may be decreased in order to prevent under delivery of the first aerosol.

In some examples, the aerosol generating system further comprises a communication unit adapted to receive a control signal, and wherein the electrical circuitry is adapted to adjust a function of the aerosol generating system in response to the control signal. In this way, the device may adapt in response to a control signal, for example received from a connected user device, for example generated by way of an application on a user's smartphone.

In some examples, adjusting a function of the aerosol generating system comprises adjusting the first delay period between activating the second aerosol generator and activating the first aerosol generator. In this way, the device may be adapted to delay the activation of the first aerosol generator with respect to the second aerosol generator according to a user preference.

In some examples, the first aerosol generator comprises a first piezoelectric transducer and the second aerosol generator comprises a second piezoelectric transducer. By using piezoelectric transducers as the first and second aerosol generators, the speed at, or reactivity with, which the first and second aerosol generator can be activated and deactivated may be improved, for example when compared to heaters which have lag times associated with heating up and cooling down.

The present disclosure may provide a method for controlling an aerosol generating system, the aerosol generating system comprising: a first aerosol generator for generating a first aerosol from a first formulation; and a second aerosol generator for generating a second aerosol from a second formulation, different from the first formulation, which may implement any one or more features disclosed herein. The method may comprise delaying the activation of the first aerosol generator with respect to the activation of the second aerosol generator for a first delay period in response to an inhalation of a user of the aerosol generating system. The method may further comprise measuring an inhalation duration of the user over a series of inhalations; determining an optimal first delay period for delaying the activation of the first aerosol generator with respect to the activation of the second aerosol generator based on an average inhalation duration of the user; and delaying the activation of the first aerosol generator with respect to the activation of the second aerosol generator for the optimal first delay period The present disclosure may provide electrical circuitry and/or a computer program configured to cause an aerosol generating apparatus/system to perform any method or method step disclosed herein. A computer readable medium comprising the computer program is also disclosed.

In a third aspect the present disclosure provides aerosol generating system that comprises a first aerosol generator for generating a first aerosol from a first formulation and a second aerosol generator for generating a second aerosol from a second formulation, different from the first formulation.

The aerosol generating system further comprises electrical circuitry, wherein the electrical circuitry is adapted to: modulate the activation of the first aerosol generator and the second aerosol generator over a single inhalation period in response to an inhalation of a user of the aerosol generating system.

In other words, as the user inhales, the aerosol generating system is adapted to modulate the activation of the first and second aerosol generators over the course of the inhalation.

Put another way, the aerosol generating system may be adapted to modulate the generation and delivery of the first and second aerosols over the course of an inhalation.

Modulation of the generation of the first and second aerosols may improve the user perception of the first aerosol and/or the second aerosol on a per inhalation basis as described in further detail in the examples provided below.

In some examples, the first formulation comprises a nicotine formulation. In some examples, the second formulation comprises a flavour formulation. In this way, the device may be adapted to modulate generating of an aerosol containing only nicotine formulation and an aerosol containing only flavour formulation in order to alter and improve the users perception of the first and/or second aerosol.

Modulating the activation of the first aerosol generator and the second aerosol generator over the single inhalation period comprises one or more of: alternating an activation state of the first and second aerosol generators over the single inhalation period.

In some embodiments modulating the activation of the first aerosol generator and the second aerosol generator over the single inhalation period further comprises delaying the activation state of one of the first and second aerosol generators with respect to another of the first and second aerosol generators over the single inhalation period.

By alternating an activation state of the first and second aerosol generators over the single inhalation period, the user's perception of the aerosol being inhaled, and in particular, the user perception of the second aerosol may be improved.

More specifically, the user perception of the second aerosol may be improved by creating a change in the composition of the aerosol being inhaled. For example, if a user takes several inhalations of aerosol containing a consistent amount of both the first and the second aerosol, the user may become used to the presence of the second aerosol, and their perception of the second aerosol may decrease. However, by providing an inhalation comprising only the first aerosol, and absent the second aerosol, for one or more portions of the inhalation and only the second aerosol, and absent the first aerosol, for one or more portions of the inhalation, the user will perceive an increase in the intensity of the second aerosol.

When referring to perceived changes in the aerosol by the user, it is to be understood that each inhalation comprising both the first aerosol and the second aerosol may be substantially identical, i.e., may comprise the same mixture, or composition, of first and second aerosols. However, when provided in the context of only providing one of the two aerosols at a time in an alternating pattern, as opposed to providing both aerosols at the same time, each individual aerosol will be more noticeable to the user. Therefore, by providing an occasional absence of the second aerosol, e.g., the flavour, the user's perception of the second aerosol will be renewed or refreshed.

By delaying the activation state of one of the first and second aerosol generators with respect to another of the first and second aerosol generators over the single inhalation period, the device may improve the user perception of the aerosol by masking the one aerosol with another aerosol. For example, the first aerosol, and in particular the taste of the first aerosol which may comprise the nicotine formulation, may be masked, covered or blocked using the second aerosol, which may comprise the flavour formulation, by starting to provide the second aerosol to the user before the first aerosol. After the delay period, the first and second aerosols may be delivered to the user simultaneously for the remainder of the inhalation.

In some examples, alternating the activation state of the first and second aerosol generators over the single inhalation period comprises one or more of: alternating the activation of the first aerosol generator and the second aerosol; alternating the activation of only the first aerosol generator and both the first aerosol generator and the second aerosol generator; and alternating the activation of only the second aerosol generator and both the first aerosol generator and the second aerosol generator.

In this way, the user perception of the first aerosol or the second aerosol may be improved by creating a change in the composition of the aerosol being inhaled. For example, if a user takes several inhalations of aerosol containing both the first and the second aerosol, the user may become used to the presence of both the second aerosol, and their perception of the first aerosol, for example the presence of nicotine, and the second aerosol, for example the flavour, may decrease. However, by providing portions of an inhalation that are absent one of the first or second aerosols, the subsequent portion of the inhalation comprising that given aerosol will provide the user with a perceived increase in the amount of said first or second aerosol present in the mixture. Therefore, the user perception of the flavour or nicotine may be kept fresh.

In some examples, the electrical circuitry is further adapted to: measure an inhalation duration of the user over a series of inhalations; determine an optimal alternation frequency for alternating between activation states of the first and second aerosol generators based on an average inhalation duration of the user; and alternating the activation states of the first and second aerosol generators at the optimal alternation frequency. In this way, the device may adapt to the user's typical inhalation period over time in order to determine the optimal alternation frequency to cause a change in the user's perception of the first and/or second aerosols, whilst also delivering a sufficient amount of the first and second aerosols within the typical inhalation period. For example, for a user with a longer typical inhalation period, the frequency of alternation between the activation states of the first and second aerosol generators may be reduced, such that a respective activation period of the first and second aerosol generators may be increased. For example, for a user with a shorter typical inhalation period, the frequency of alternation between the activation states of the first and second aerosol generators may be increased, such that a respective activation period of the first and second aerosol generators may be decreased.

In some examples, delaying the activation state of one of the first and second aerosol generators with respect to another of the first and second aerosol generators over the single inhalation period comprises one or more of: delaying the activation of the first aerosol generator with respect to the activation of the second aerosol generator for a first delay period during the single inhalation period; and delaying the activation of the second aerosol generator with respect to the activation of the first aerosol generator for a second delay period during the single inhalation period.

By delaying the activation of the first aerosol generator with respect to the activation of the second aerosol generator for a first delay period during the single inhalation period, the taste of the first aerosol, for example the nicotine formulation, may be masked using the second aerosol, for example the flavour formulation, by starting to provide the second aerosol to the user before the first aerosol. After the first delay period, the first and second aerosols may be delivered to the user simultaneously for the remainder of the inhalation.

By delaying the activation of the second aerosol generator with respect to the activation of the first aerosol generator for a second delay period during the single inhalation period, the user may be provided with a perceived change in the intensity of the second aerosol, for example the flavour formulation, thereby improving the user's perception of the second aerosol. After the second delay period, the first and second aerosols may be delivered to the user simultaneously for the remainder of the inhalation.

In some examples, the electrical circuitry is further adapted to: measure an inhalation duration of the user over a series of inhalations; determine an optimal delay period for delaying the activation of one of the first and second aerosol generators with respect to another of the first and second aerosol generators over the single inhalation period; and delaying the activation of the first aerosol generator with respect to the activation of the second aerosol generator for the optimal delay period during the single inhalation period; or delaying the activation of the second aerosol generator with respect to the activation of the first aerosol generator for the optimal delay period during the single inhalation period.

In this way, the device may adapt to the user's typical inhalation period over time in order to determine an optimal first delay period such that the first aerosol is masked by the second aerosol, but a sufficient amount of first aerosol is also delivered within the typical inhalation period and/or an optimal second delay period such that the user perception of the second aerosol is improved, but a sufficient amount of second aerosol is also delivered.

In some examples, the aerosol generating system further comprises a pressure sensor adapted to sense an inhalation of the user and send an inhalation signal to the electrical circuitry in response to sensing the inhalation of the user, and wherein the electrical circuitry is adapted to activate the first and second aerosol generators in response to the inhalation signal. In this way, the device may respond automatically to an inhalation of the user based on the pressure sensor, rather than requiring an additional user input.

In some examples, the electrical circuitry is further adapted to: determine an inhalation strength based on the inhalation signal; and adjust the modulation of the activation of the first aerosol generator and the second aerosol generator based on the inhalation strength. In this way, the device may adapt the modulation of the provision of the first and second aerosols according to the inhalation strength of the user. For example, for a strong inhalation, i.e., an inhalation with a strength above a predetermined threshold, the delivery of the first aerosol may be reduced by the modulation in order to prevent over delivery of the first aerosol. In another example, for a weak inhalation, i.e., an inhalation with a strength below a predetermined threshold, the delivery of the first aerosol may be increased by the modulation in order to prevent under delivery of the first aerosol.

In some examples, the aerosol generating system further comprises a communication unit adapted to receive a control signal, and wherein the electrical circuitry is adapted to adjust a function of the aerosol generating system in response to the control signal, and wherein adjusting a function of the aerosol generating system comprises adjusting the modulation of the activation of the first aerosol generator and the second aerosol generator based on the control signal. In this way, the device may adapt in response to a control signal, for example received from a connected user device, for example generated by way of an application on a user's smartphone.

In some examples, the first aerosol generator comprises a first piezoelectric transducer and the second aerosol generator comprises a second piezoelectric transducer. In this way, the device may generate the first and second aerosols by way of ultrasonic cavitation. By using piezoelectric transducers as the first and second aerosol generators, the speed at, or reactivity with, which the first and second aerosol generator can be activated and deactivated may be improved, for example when compared to heaters which have lag times associated with heating up and cooling down.

The present disclosure may provide a method for controlling an aerosol generating system, the aerosol generating system comprising: a first aerosol generator for generating a first aerosol from a first formulation; and a second aerosol generator for generating a second aerosol from a second formulation, different from the first formulation, which may implement any one or more features disclosed herein. The method may comprise modulating the activation of the first aerosol generator and the second aerosol generator over a single inhalation period in response to an inhalation of a user of the aerosol generating system.

Modulating the activation of the first aerosol generator and the second aerosol generator over the single inhalation period may comprise one or more of: alternating an activation state of the first and second aerosol generators over the single inhalation period; and delaying the activation state of one of the first and second aerosol generators with respect to another of the first and second aerosol generators over the single inhalation period.

Alternating the activation state of the first and second aerosol generators over the single inhalation period may comprise one or more of: alternating the activation of the first aerosol generator and the second aerosol; alternating the activation of only the first aerosol generator and both the first aerosol generator and the second aerosol generator; and alternating the activation of only the second aerosol generator and both the first aerosol generator and the second aerosol generator.

Delaying the activation state of one of the first and second aerosol generators with respect to another of the first and second aerosol generators over the single inhalation period may comprise one or more of: delaying the activation of the first aerosol generator with respect to the activation of the second aerosol generator for a first delay period during the single inhalation period; and delaying the activation of the second aerosol generator with respect to the activation of the first aerosol generator for a second delay period during the single inhalation period.

The present disclosure may provide electrical circuitry and/or a computer program configured to cause an aerosol generating apparatus/system to perform any method or method step disclosed herein. A computer readable medium comprising the computer program is also disclosed.

In a fourth aspect the present disclosure provides an aerosol generating system that comprises a first aerosol generator for generating a first aerosol from a first formulation and a second aerosol generator for generating a second aerosol from a second formulation, different from the first formulation.

The aerosol generating system further comprises electrical circuitry adapted to alternate the activation of the first aerosol generator and the second aerosol generator over a series of inhalations of a user of the aerosol generating system, wherein alternating the activation of the first aerosol generator and the second aerosol generator comprises switching between activating the first/second aerosol generator and activating the second/first aerosol generator on consecutive inhalations.

In other words, the aerosol generating system may be adapted to alternate between generating the first aerosol alone and generating the second aerosol alone or alternate between generating the second aerosol alone and generating the first aerosol alone.

Put another way, the aerosol generating system may be adapted to alternate between generating different pure aerosols, i.e., aerosols generated from a single aerosol precursor liquid.

In this way, the device may alternate between providing different aerosols to the user over a series of multiple inhalations. By alternating between providing only the first aerosol and only the second aerosol, the user's perception of the respective aerosol being inhaled, i.e., the user perception of the first aerosol and the second aerosol, in turn, may be improved.

More specifically, the user perception of the first aerosol may be improved by creating a change in the composition of the aerosol being inhaled between inhalations. For example, if a user were to take several inhalations of aerosol containing both the first and the second aerosol, the user may become used to the presence of the first and the second aerosol, and their perception of both the first aerosol and the second aerosol may decrease. However, by providing an inhalation comprising only the second aerosol, and absent the first aerosol, the subsequent inhalation comprising only the first aerosol will provide the user with a perceived increase in intensity of the first aerosol.

Similarly, the user perception of the second aerosol may be improved by creating a change in the composition of the aerosol being inhaled between inhalations. For example, if a user were to take several inhalations of aerosol containing both the first and the second aerosol, the user may become used to the presence of the first and the second aerosol, and their perception of both the first aerosol and the second aerosol may decrease. However, by providing an inhalation comprising only the first aerosol, and absent the second aerosol, the subsequent inhalation comprising only the second aerosol will provide the user with a perceived increase in intensity of the second aerosol.

When referring to perceived changes in the aerosol by the user, it is to be understood that each inhalation comprising the first aerosol or the second aerosol may be substantially identical, i.e., may comprise the same amount of first or second aerosols. However, when provided in the context of immediately following an inhalation comprising only the first aerosol, the presence of the second aerosol will be more noticeable to the user. Similarly, when provided in the context of immediately following an inhalation comprising only the second aerosol, the presence of the first aerosol will be more noticeable to the user. Therefore, by providing an occasional absence of the second aerosol, or the first aerosol, the user's perception of the second aerosol, or the first aerosol, will be renewed or refreshed.

In some examples, the first formulation comprises a nicotine formulation. In some examples, the second formulation comprises a flavour formulation. In this way, the device may be adapted to switch between delivering the first aerosol, comprising the nicotine formulation, and delivering the second aerosol, comprising the flavour formulation on consecutive inhalations. In this way, the device may be adapted to alternate between generating an aerosol containing only nicotine formulation and an aerosol containing only flavour formulation. As described above, in this way the device may be adapted to improve the user perception of both the intensity of the nicotine in the first aerosol and the intensity of the flavour of the second aerosol over a series of inhalations. In this way, the user perception of the nicotine and flavour formulations may be kept fresh over a longer period of time.

In some examples, on startup of the aerosol generating system, the electrical circuitry is adapted to initially activate the second aerosol generator in response to a user inhalation. In this way, the device may provide the user with the second aerosol for the first inhalation of the user and then switch to providing the user with the first aerosol for the subsequent, second inhalation. For example, when the second aerosol comprises a flavour formulation, the flavour formulation may be delivered to the user in the inhalation before the first aerosol, comprising the nicotine formulation, is delivered to the user in order to mask, block or cover the taste of the first aerosol.

In some examples, the electrical circuitry is further adapted to: measure an inhalation duration of the user over a series of inhalations; determine a first average inhalation duration for inhalations when the first aerosol generator is active; determine a second average inhalation duration for inhalations when the second aerosol generator is active; compare the first average inhalation duration and the second average inhalation duration; and if the first average inhalation duration is greater than the second average inhalation duration, reduce a first activation period of the first aerosol generator, wherein the first activation period is a portion of the first average inhalation time; and if the second average inhalation duration is greater than the first average inhalation duration, reduce a second activation period of the second aerosol generator, wherein the second activation period is a portion of the second average inhalation time.

In this way, the device may balance the delivery of the first aerosol and the second aerosol over the series of inhalations by controlling the activation periods of the first and second aerosol generators within the inhalation periods of the user. Thus, the device may prevent the depletion of one of the liquid aerosol precursors before the other liquid aerosol precursor, for example where the user inhales for longer on average when the second aerosol generator is activated.

In some examples, the electrical circuitry is further adapted to: determine an elapsed time since an inhalation last occurred; and switch to activating the second aerosol generator for a next inhalation of the user of the aerosol generating system based on the elapsed time.

In this way, the device may be adapted to reset the alternation between activating only the first aerosol generator and activating only the second aerosol generator after a given period of inactivity, i.e., the elapsed time since an inhalation last occurred. The user's perception of the second aerosol will be refreshed naturally after a period of time following an inhalation. Thus, by switching to activating the second aerosol generator after a given period of inactivity, the user will be provided with a perceived change in intensity of the second aerosol, e.g., the flavour, naturally. Further, in this way, the device may be prevented from generating only the first aerosol in response to the first inhalation of a user in an inhalation session, thereby preventing the first inhalation of a session from lacking the second aerosol, which may for example be the flavour.

In some examples, switching to activating the first aerosol generator based on the elapsed time comprises: determining whether the elapsed time exceeds a predetermined period; if the elapsed time exceeds the predetermined period, switching to activating the second aerosol generator; and if the elapsed time exceeds the predetermined period, maintaining a current activation state of the first and second aerosol generators.

In this way, the device may recognise a new inhalation session, and respond by activating the second aerosol generator as the user perception of the second aerosol will already be refreshed by virtue of the elapsed time since an inhalation last occurred being sufficiently long.

In some examples, the aerosol generating system further comprises a pressure sensor adapted to sense an inhalation of the user and send an inhalation signal to the electrical circuitry in response to sensing the inhalation of the user, and wherein the electrical circuitry is adapted to activate one of the first and second aerosol generators in response to the inhalation signal. In this way, the device may respond automatically to an inhalation of the user based on the pressure sensor, rather than requiring an additional user input.

In some examples, the electrical circuitry is further adapted to: determine an inhalation strength based on the inhalation signal over a series of inhalations; determine a first average inhalation strength for inhalations when the first aerosol generator is active; determine a second average inhalation strength for inhalations when the second aerosol generator is active; compare the first average inhalation strength and the second average inhalation strength; and if the first average inhalation strength is greater than the second average inhalation strength, reduce an activation period of the first aerosol generator; and if the second average inhalation strength is greater than the first average inhalation strength, reduce an activation period of the second aerosol generator.

In this way, the device may balance the delivery of the first aerosol and the second aerosol over the series of inhalations by controlling the activation periods of the first and second aerosol generators within the inhalation periods of the user. Thus, the device may prevent the depletion of one of the liquid aerosol precursors before the other liquid aerosol precursor, for example where the user performs a stronger inhalation on average when the second aerosol generator is activated.

In some examples, the aerosol generating system further comprises a communication unit adapted to receive a control signal, and wherein the electrical circuitry is adapted to adjust a function of the aerosol generating system in response to the control signal. In this way, the device may adapt in response to a control signal, for example received from a connected user device, for example generated by way of an application on a user's smartphone.

In some examples, adjusting a function of the aerosol generating system comprises switching from a current activation state of the first and second aerosol generators to activating one of the first and second aerosol generators based on the control signal. In this way, the device may switch from activating only the first aerosol generator or activating only the second aerosol generator according to a user preference.

In some examples, the first aerosol generator comprises a first piezoelectric transducer and the second aerosol generator comprises a second piezoelectric transducer. In this way, the device may generate the first and second aerosols by way of ultrasonic cavitation. By using piezoelectric transducers as the first and second aerosol generators, the speed at, or reactivity with, which the first and second aerosol generator can be activated and deactivated may be improved, for example when compared to heaters which have lag times associated with heating up and cooling down.

The present disclosure may provide a method of generating an aerosol an aerosol generating system, the aerosol generating system comprising: a first aerosol generator for generating a first aerosol from a first formulation; and a second aerosol generator for generating a second aerosol from a second formulation, different from the first formulation, which may implement any one or more features disclosed herein. The method may comprise alternating the activation of the first aerosol generator and the second aerosol generator over a series of inhalations of a user of the aerosol generating system, wherein alternating the activation of the first aerosol generator and the second aerosol generator comprises switching between activating the first/second aerosol generator and activating the second/first aerosol generator on consecutive inhalations.

The method may further comprise: measuring an inhalation duration of the user over a series of inhalations; determining a first average inhalation duration for inhalations when the first aerosol generator is active; determining a second average inhalation duration for inhalations when the second aerosol generator is active; comparing the first average inhalation duration and the second average inhalation duration; and if the first average inhalation duration is greater than the second average inhalation duration, reducing a first activation period of the first aerosol generator, wherein the first activation period is a portion of the first average inhalation time; and if the second average inhalation duration is greater than the first average inhalation duration, reducing a second activation period of the second aerosol generator, wherein the second activation period is a portion of the second average inhalation time.

In some examples, the aerosol generating system further comprises a pressure sensor adapted to sense an inhalation of the user and send an inhalation signal to the electrical circuitry in response to sensing the inhalation of the user, and wherein the electrical circuitry is adapted to activate one of the first and second aerosol generators in response to the inhalation signal. In this case, the method may further comprise determining an inhalation strength based on the inhalation signal over a series of inhalations; determining a first average inhalation strength for inhalations when the first aerosol generator is active; determining a second average inhalation strength for inhalations when the second aerosol generator is active; comparing the first average inhalation strength and the second average inhalation strength; and if the first average inhalation strength is greater than the second average inhalation strength, reducing an activation period of the first aerosol generator; and if the second average inhalation strength is greater than the first average inhalation strength, reducing an activation period of the second aerosol generator.

The present disclosure may provide electrical circuitry and/or a computer program configured to cause an aerosol generating apparatus/system to perform any method or method step disclosed herein. A computer readable medium comprising the computer program is also disclosed.

In embodiments, the electrical circuitry is implemented as one or more processors, which are configured to implement the disclosed steps, e.g. as the controller. The processors may execute program code stored on electronic memory and/or may execute logic, e.g. as a logic array, gate array, structured gate array.

The preceding summary is provided for purposes of summarizing some examples to provide a basic understanding of aspects of the subject matter described herein. Accordingly, the above-described features should not be construed to narrow the scope or spirit of the subject matter described herein in any way. Moreover, the above and/or proceeding examples may be combined in any suitable combination to provide further examples, except where such a combination is clearly impermissible or expressly avoided. Other features, aspects, and advantages of the subject matter described herein will become apparent from the following text and the accompanying drawings.

### BRIEF DESCRIPTION OF THE FIGURES

Aspects, features and advantages of the present disclosure will become apparent from the following description of examples in reference to the appended drawings in which like numerals denote like elements.
Fig. 1 is a block system diagram showing an example aerosol generating apparatus.
Fig. 2 is a block system diagram showing an example implementation of the apparatus of Fig. 1, where the aerosol generating apparatus is configured to generate aerosol from a liquid precursor.
Figs. 3A and 3B are schematic diagrams showing an example implementation of the apparatus of Fig. 2.
Fig. 4 is a block system diagram showing an example system for managing an aerosol generating apparatus.
Fig. 5 shows an example of a circuit for modelling the behaviour of an exemplary piezoelectric transducer.
Fig. 6 shows a portion of an exemplary driving circuit using an H-bridge.
Fig. 7 shows an example of an improved driving circuit for driving a piezoelectric transducer.
Fig. 8 shows a schematic representation of an aerosol generating system according to an aspect of the invention.
Fig. 9 shows a method for controlling an aerosol generating system according to an aspect of the invention.
Fig. 10 shows a schematic representation of the driving scheme for two aerosol generating units according to the method shown in Fig. 9.
Fig. 11 shows a method for controlling an aerosol generating system according to an aspect of the invention.
Fig. 12 shows a schematic representation of the driving scheme for two aerosol generating units according to the method shown in Fig. 11.
Fig. 13 shows a method for controlling an aerosol generating system according to an aspect of the invention.
Fig. 14 shows a schematic representation of the driving scheme for two aerosol generating units according to the method shown in Fig. 14.
Fig. 15 shows a method for controlling an aerosol generating system according to an aspect of the invention.
Fig. 16 shows a schematic representation of the driving scheme for two aerosol generating units according to the method shown in Fig. 15.

### DETAILED DESCRIPTION OF EMBODIMENTS

Before describing several examples implementing the present disclosure, it is to be understood that the present disclosure is not limited by specific construction details or process steps set forth in the following description and accompanying drawings. Rather, it will be apparent to those skilled in the art having the benefit of the present disclosure that the systems, apparatuses and/or methods described herein could be embodied differently and/or be practiced or carried out in various alternative ways.

Unless otherwise defined herein, scientific and technical terms used in connection with the presently disclosed inventive concept(s) shall have the meanings that are commonly understood by those of ordinary skill in the art, and known techniques and procedures may be performed according to conventional methods well known in the art and as described in various general and more specific references that may be cited and discussed in the present specification.

Any patents, published patent applications, and non-patent publications mentioned in the specification are hereby incorporated by reference in their entirety.

All examples implementing the present disclosure can be made and executed without undue experimentation in light of the present disclosure. While particular examples have been described, it will be apparent to those of skill in the art that variations may be applied to the systems, apparatus, and/or methods and in the steps or in the sequence of steps of the methods described herein without departing from the concept, spirit, and scope of the inventive concept(s). All such similar substitutions and modifications apparent to those skilled in the art are deemed to be within the spirit, scope, and concept of the inventive concept(s) as defined by the appended claims.

The use of the term "a" or "an" in the claims and/or the specification may mean "one," as well as "one or more," "at least one," and "one or more than one." As such, the terms "a," "an," and "the," as well as all singular terms, include plural referents unless the context clearly indicates otherwise. Likewise, plural terms shall include the singular unless otherwise required by context.

The use of the term "or" in the present disclosure (including the claims) is used to mean an inclusive "and/or" unless explicitly indicated to refer to alternatives only or unless the alternatives are mutually exclusive. For example, a condition "A or B" is satisfied by any of the following: A is true (or present) and B is false (or not present), A is false (or not present) and B is true (or present), and both A and B are true (or present).

As used in this specification and claim(s), the words "comprising, "having," "including," or "containing" (and any forms thereof, such as "comprise" and "comprises," "have" and "has," "includes" and "include," or "contains" and "contain," respectively) are inclusive or open-ended and do not exclude additional, unrecited elements or method steps.

Unless otherwise explicitly stated as incompatible, or the physics or otherwise of the embodiments, examples, or claims prevent such a combination, the features of examples disclosed herein, and of the claims, may be integrated together in any suitable arrangement, especially ones where there is a beneficial effect in doing so. This is not limited to only any specified benefit, and instead may arise from an "ex post facto" benefit. This is to say that the combination of features is not limited by the described forms, particularly the form (e.g. numbering) of example(s), embodiment(s), or dependency of claim(s). Moreover, this also applies to the phrase "in one embodiment," "according to an embodiment," and the like, which are merely a stylistic form of wording and are not to be construed as limiting the following features to a separate embodiment to all other instances of the same or similar wording. This is to say, a reference to 'an,' 'one,' or 'some' embodiment(s) may be a reference to any one or more, and/or all embodiments, or combination(s) thereof, disclosed. Also, similarly, the reference to "the" embodiment may not be limited to the immediately preceding embodiment. Further, all references to one or more embodiments or examples are to be construed as non-limiting to the claims.

The present disclosure may be better understood in view of the following explanations, wherein the terms used that are separated by "or" may be used interchangeably:
As used herein, an "aerosol generating apparatus" (or "electronic(e)-cigarette") may be an apparatus configured to deliver an aerosol to a user for inhalation by the user. The apparatus may additionally/alternatively be referred to as a "smoking substitute apparatus", if it is intended to be used instead of a conventional combustible smoking article. As used herein a combustible "smoking article" may refer to a cigarette, cigar, pipe or other article, that produces smoke (an aerosol comprising solid particulates and gas) via heating above the thermal decomposition temperature (typically by combustion and/or pyrolysis). An aerosol generated by the apparatus may comprise an aerosol with particle sizes of 0.2 - 7 microns, such as between 2 - 3 microns, or less than 10 microns, or less than 7 microns, or less than 3 microns, or less than 2 microns. This particle size may be achieved by control of one or more of: driving parameters of the ultrasonic generator; flow properties including turbulence and velocity. The generation of aerosol by the aerosol generating apparatus may be controlled by an input device. The input device may be configured to be user-activated, and may for example include or take the form of an actuator (e.g. actuation button) and/or an airflow sensor.

Each occurrence of the aerosol generating apparatus being caused to generate aerosol for a period of time (which may be variable) may be referred to as an "activation" of the aerosol generating apparatus. The aerosol generating apparatus may be arranged to allow an amount of aerosol delivered to a user to be varied per activation (as opposed to delivering a fixed dose of aerosol), e.g. by activating an aerosol generating unit of the apparatus for a variable amount of time, e.g. based on the strength/duration of a draw of a user through a flow path of the apparatus (to replicate an effect of smoking a conventional combustible smoking article).

The aerosol generating apparatus may be portable. As used herein, the term "portable" may refer to the apparatus being for use when held by a user.

As used herein, an "aerosol generating system" may be a system that includes an aerosol generating apparatus and optionally other circuitry/components associated with the function of the apparatus, e.g. one or more external devices and/or one or more external components (here "external" is intended to mean external to the aerosol generating apparatus). As used herein, an "external device" and "external component" may include one or more of a: a charging device, a mobile device (which may be connected to the aerosol generating apparatus, e.g. via a wireless or wired connection); a networked-based computer (e.g. a remote server); a cloud-based computer; any other server system.

An example aerosol generating system may be a system for managing an aerosol generating apparatus. Such a system may include, for example, a mobile device, a network server, as well as the aerosol generating apparatus.

As used herein, an "aerosol" may include a suspension of liquid droplets of precursor. An aerosol may include one or more components of the precursor.

As used herein, a "precursor" may include one or more of a: liquid; and a gel. The precursor may be processed by an aerosol generating unit of an aerosol generating apparatus to generate an aerosol. The precursor may include one or more of: an active component; a carrier; a flavouring. The active component may include one or more of nicotine; caffeine; a cannabidiol oil; a non-pharmaceutical formulation, e.g. a formulation which is not for treatment of a disease or physiological malfunction of the human body. The active component may be carried by the carrier, which may be a liquid, including propylene glycol and/or glycerine. The term "flavouring" may refer to a component that provides a taste and/or a smell to the user. The flavouring may include one or more of: Ethylvanillin (vanilla); menthol, Isoamyl acetate (banana oil); or other. The precursor may include a carrier; a flavouring.

As used herein, a "storage portion" may be a portion of the apparatus adapted to store the precursor. It may be implemented as fluid-holding reservoir depending on the implementation of the precursor as defined above.

As used herein, a "flow path" may refer to a path or enclosed passageway through an aerosol generating apparatus, e.g. for delivery of an aerosol to a user. The flow path may be arranged to receive aerosol from an aerosol generating unit. When referring to the flow path, upstream and downstream may be defined in respect of a direction of flow in the flow path, e.g. with an outlet being downstream of an inlet.

As used herein, a "delivery system" may be a system operative to deliver an aerosol to a user. The delivery system may include a mouthpiece and a flow path. The delivery system may be at least partly within the aerosol generating component.

As used herein, a "flow" may refer to a flow in a flow path. A flow may include aerosol generated from the precursor. The flow may include air, which may be induced into the flow path via a puff by a user.

As used herein, a "puff' (or "inhale" or "draw") by a user may refer to expansion of lungs and/or oral cavity of a user to create a pressure reduction that induces flow through the flow path.

As used herein, an "aerosol generating unit" may refer to a device configured to generate an aerosol from a precursor. The aerosol generating unit may include a unit to generate an aerosol directly from the precursor (e.g. an atomiser including an ultrasonic system). A plurality of aerosol generating units to generate a plurality of aerosols (for example, from a plurality of different aerosol precursors) may be present in an aerosol generating apparatus.

As used herein, an "ultrasonic generator" may refer to a piezoelectric transducer capable of vibrating at ultrasonic frequencies, i.e., at frequencies greater than 20kHz. In some examples, the piezoelectric transducer may be capable of vibrating at even higher frequencies, e.g., at frequencies of 100 kHz or above, 500 kHz or above, 1 MHz or more, 2 MHz or more, 5 MHz or more, or 10 MHz or more. The piezoelectric transducer may be adapted to vibrate in response to a driving signal, and in particular adapted to vibrate at the frequency of the driving signal. In some examples, the driving signal may be generated by direct digital synthesis (DDS).

As used herein, a "piezoelectric transducer" may refer to an ultrasonic transducer comprising a piezoelectric crystal, which generates a mechanical strain internally in response to an electric field. A rapidly changing electric field, such as an ultrasonic frequency driving signal, results in rapidly changing mechanical strain within the piezoelectric crystal causing it to vibrate. The piezoelectric transducer will have an aerosolisation surface from which the aerosol is generated. The aerosolisation surface typically faces into an aerosolisation chamber.

As used herein, an "**aerosol generating component**" may refer to a component that includes an aerosol precursor. The component may include an aerosol generating unit e.g. it may be arranged as a cartomizer. The component may include a mouthpiece. The component may include an information carrying medium. The component may include a storage portion, e.g. a reservoir or tank, for storage of the aerosol precursor.

With liquid or gel implementations of the aerosol precursor, e.g. an e-liquid, the component may be referred to as a "capsule" or a "pod" or an "e-liquid consumable". In some embodiments, the aerosol precursor component may be affixed to the device body to form the aerosol generating apparatus. In these embodiments, the reservoir/tank may be refillable.

The aerosol generating component e.g. the capsule, pod, or consumable may be for releasable coupling to a device body to form the aerosol generating apparatus.

The device body may comprise a power supply for powering the aerosol generating unit.

As used herein, an "information carrying medium" may include one or more arrangements for storage of information on any suitable medium. Examples include: a computer readable medium; a Radio Frequency Identification (RFID) transponder; codes encoding information, such as optical (e.g. a bar code or QR code) or mechanically read codes (e.g. a configuration of the absence or presents of cutouts to encode a bit, through which pins or a reader may be inserted).

As used herein, "electrical circuitry" may refer to one or more electrical components, examples of which may include: an Application Specific Integrated Circuit (ASIC) or other programable logic; electronic/electrical componentry (which may include combinations of transistors, resistors, capacitors, inductors etc); one or more processors (e.g. the circuitry structure of the processor); a non-transitory memory (e.g. implemented by one or more memory devices), that may store one or more software or firmware programs; a combinational logic circuit; interconnection of the aforesaid. The electrical circuitry may be located entirely at the apparatus (e.g., in the device/main body comprising the power supply), or distributed between the apparatus and/or on one or more external devices in communication with the apparatus, e.g. as part of a system. The electrical circuitry may comprise a controller. The controller may be configured to carry out any of the methods described herein.

As used herein, a "processing resource" (or "processor" or "controller") may refer to one or more units for processing data, examples of which may include an ASIC, microcontroller, FPGA, microprocessor, digital signal processor (DSP) capability, state machine or other suitable component. A processing resource may be configured to execute a computer program, e.g. which may take the form of machine readable instructions, which may be stored on a non-transitory memory and/or programmable logic. The processing resource may have various arrangements corresponding to those discussed for the circuitry, e.g. on-board and/or off board the apparatus as part of the system. As used herein, any machine executable instructions, or computer readable media, may be configured to cause a disclosed method to be carried out, e.g. by an aerosol generating apparatus or system as disclosed herein, and may therefore be used synonymously with the term method.

As used herein, an "external device" (or "peripheral device") may include one or more electronic components external to an aerosol generating apparatus. Those components may be arranged at the same location as the aerosol generating apparatus or remote from the apparatus. An external device may comprise electronic computer devices including: a smartphone; a PDA; a video game controller; a tablet; a laptop; or other like device.

As used herein, a "computer readable medium/media" (or "memory" or "data storage") may include any medium capable of storing a computer program, and may take the form of any conventional non-transitory memory, for example one or more of: random access memory (RAM); a CD; a hard drive; a solid state drive; a memory card; a DVD. The memory may have various arrangements corresponding to those discussed for the circuitry /processor. The present disclosure includes a computer readable medium configured to cause an apparatus or system disclosed herein to perform a method as disclosed herein.

As used herein, a "communication resource" (or "communication interface") may refer to hardware and/or firmware for electronic information/data transfer. The communication resource may be configured for wired communication ("wired communication resources") or wireless communication ("wireless communication resource"). Wireless communication resources may include hardware to transmit and receive signals by radio and may include various protocol implementations e.g. the 802.11 standard described in the Institute of Electronics Engineers (IEEE) and Bluetooth^{™} from the Bluetooth Special Interest Group of Kirkland Wash. Wired communication resources may include; Universal Serial Bus (USB); High-Definition Multimedia Interface (HDMI) or other protocol implementations. The apparatus may include communication resources for wired or wireless communication with an external device.

As used herein, a "network" (or "computer network") may refer to a system for electronic information/data transfer between a plurality of apparatuses/devices. The network may, for example, include one or more networks of any type, which may include: a Public Land Mobile Network (PLMN); a telephone network (e.g. a Public Switched Telephone Network (PSTN) and/or a wireless network); a local area network (LAN); a metropolitan area network (MAN); a wide area network (WAN); an Internet Protocol Multimedia Subsystem (IMS) network; a private network; the Internet; an intranet.

It will be appreciated that any of the disclosed methods (or corresponding apparatuses, programs, data carriers, etc.) may be carried out by either a host or client, depending on the specific implementation (i.e. the disclosed methods/apparatuses are a form of communication(s), and as such, may be carried out from either 'point of view', i.e. in corresponding to each other fashion). Furthermore, it will be understood that the terms "receiving" and "transmitting" encompass "inputting" and "outputting" and are not limited to an RF context of transmitting and receiving electromagnetic (e.g. radio) waves. Therefore, for example, a chip or other device or component for realizing embodiments could generate data for output to another chip, device or component, or have as an input data from another chip, device, or component, and such an output or input could be referred to as "transmit" and "receive" including gerund forms, that is, "transmitting" and "receiving," as well as such "transmitting" and "receiving" within an RF context.

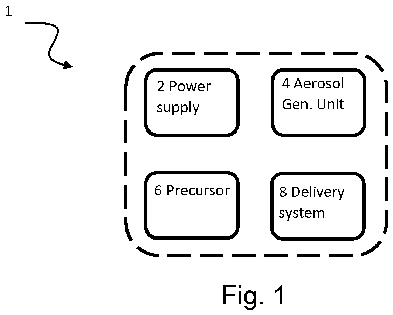

Referring to Fig. 1, an example aerosol generating apparatus 1 includes a power supply 2, for supply of electrical energy. The apparatus 1 includes an aerosol generating unit 4 that is driven by the power supply 2. The power supply 2 may include an electric power supply in the form of a battery and/or an electrical connection to an external power source. The apparatus 1 includes a precursor 6, which in use is aerosolised by the aerosol generating unit 4 to generate an aerosol. The aerosol generating unit 4 includes a piezoelectric transducer (discussed below) configured to induce, by vibration of the piezoelectric transducer i.e. vibration of an aerosolisation surface of the piezoelectric transducer, cavitation in the precursor 6. Collapse of the cavities in the precursor 6 induces a shock that propagates through the liquid precursor 6. This shock disturbs a surface of the liquid precursor 6 that interfaces with air within an aerosolisation chamber of the aerosol generating apparatus 1 (which in turn is in fluid communication with an airflow path within the aerosol generating apparatus). These disturbances take the form of ripples, also known as capillary waves, that form ligaments at the peaks of the ripples/waves, pinch off and expel droplets from the liquid precursor 6 into the airflow path, thereby aerosolising the precursor 6 to generate the aerosol. The apparatus 2 includes a delivery system 8 for delivery of the aerosol to a user.

Electrical circuitry (not shown in figure 1) may be implemented to control the interoperability of the power supply 2 and aerosol generating unit 4.

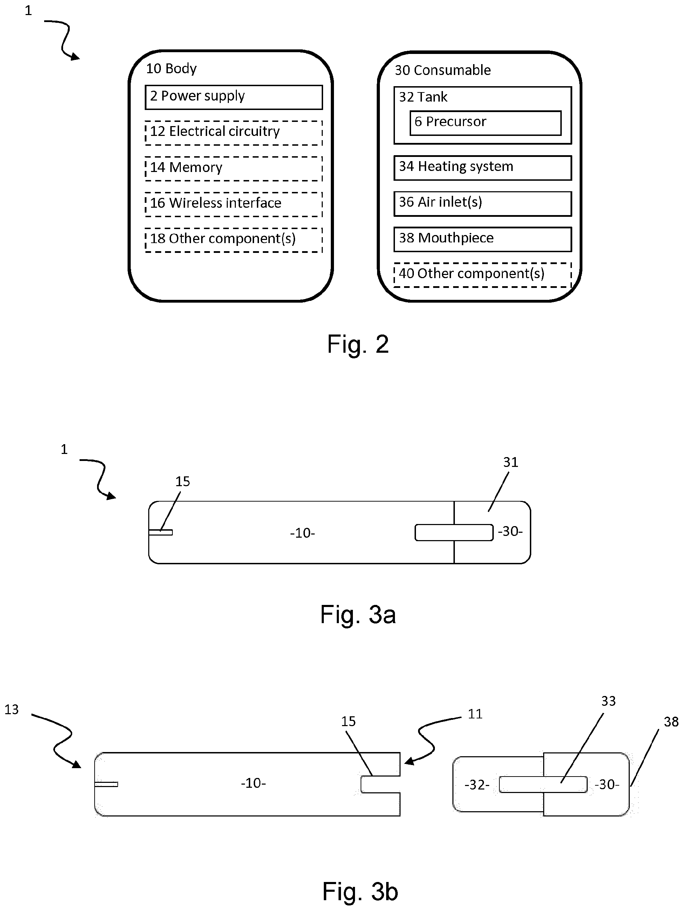

Fig. 2 shows an implementation of the apparatus 1 of Fig. 1, where the aerosol generating apparatus 1 is configured to generate aerosol from a liquid precursor.

In this example, the apparatus 1 includes a device body 10 and a consumable 30.

In this example, the body 10 includes the power supply 2. The body may additionally include any one or more of electrical circuitry 12, a memory 14, a wireless interface 16, one or more other components 18.

The electrical circuitry 12 may include a processing resource for controlling one or more operations of the body 10 and consumable 30, e.g. based on instructions stored in the memory 14.

The wireless interface 16 may be configured to communicate wirelessly with an external (e.g. mobile) device, e.g. via Bluetooth.

The other component(s) 18 may include one or more user interface devices configured to convey information to a user and/or a charging port, for example (see e.g. Fig. 3).

The consumable 30 includes a storage portion implemented here as a tank 32 which stores the liquid precursor 6 (e.g. e-liquid). The consumable 30 also includes one or more air inlets 36, and a mouthpiece 38. The consumable 30 may include one or more other components 40.

The body 10 and consumable 30 may each include a respective electrical interface (not shown) to provide an electrical connection between one or more components of the body 10 with one or more components of the consumable 30. In this way, electrical power can be supplied to components of the consumable 30, without the consumable 30 needing to have its own power supply.

The piezoelectric transducer 34 of the aerosol generating unit 4 is arranged to be in electrical contact with one or more components of the body 10. For example, the power supply 2 may be configured to provide power to the piezoelectric transducer 34. Additionally or alternatively, the piezoelectric transducer 34 may be in electrical contact/communication with one or more of the electrical circuitry 12, memory 14, wireless interface 16 or one or more of the one or more other components 18 e.g., to receive instructions to adjust an operating parameter of the piezoelectric transducer 34 and/or to transmit data indicative of the operational parameters of the piezoelectric transducer 34.

Moreover, the piezoelectric transducer 34 is arranged to be in fluid communication with the tank 32 e.g. via a wick such that the liquid precursor can be provided to the aerosolisation surface of the piezoelectric transducer 34.

In use, a user may activate the aerosol generating apparatus 1 when inhaling through the mouthpiece 38, i.e. when performing a puff. The puff, performed by the user, may initiate a flow through a flow path in the consumable 30 which extends from the air inlet(s) 36 to the mouthpiece 38 via a region (i.e. an aerosolisation chamber) in proximity to the piezoelectric transducer 34.

Activation of the aerosol generating apparatus 1 may be initiated, for example, by an airflow sensor in the body 10 which detects airflow in the aerosol generating apparatus 1 (e.g. caused by a user inhaling through the mouthpiece), or by actuation of an actuator included in the body 10. Upon activation, the electrical circuitry 12 (e.g. under control of the processing resource) may supply electrical energy from the power supply 2 to the piezoelectric transducer 34 of the aerosol generating unit 4, which may cause the piezoelectric transducer 34 to induce cavitation in the liquid precursor 6 drawn from the tank so as to produce an aerosol which is carried by the flow out of the mouthpiece 38.

In some examples, the consumable may include a wick, wherein a first portion of the wick extends into the tank 32 in order to draw liquid precursor 6 out from the tank 32 and wherein a second portion of the wick is arranged to convey the drawn liquid precursor 6 to the aerosolisation surface piezoelectric transducer 34of the aerosol generating unit 4.

In this example, the delivery system 8 is provided by the above-described flow path and mouthpiece 38.

In variant embodiments (not shown), any one or more of the precursor 6, air inlet(s) 36 and mouthpiece 38, may be included in the body 10. For example, the mouthpiece 36 may be included in the body 10 with the precursor 6 arranged as a separable cartomizer.

Figs. 3A and 3B show an example implementation of the aerosol generating apparatus 1 of Fig. 2. In this example, the consumable 30 is implemented as a capsule/pod, which is shown in Fig. 3A as being physically coupled to the body 10, and is shown in Fig. 3B as being decoupled from the body 10.

In this example, the body 10 and the consumable 30 are configured to be physically coupled together by pushing the consumable 30 into an aperture in a top end 11 the body 10, with the consumable 30 being retained in the aperture via an interference fit.

In other examples (not shown), the body 10 and the consumable 30 could be physically coupled together in other ways, e.g. by screwing one onto the other, through a bayonet fitting, or through a snap engagement mechanism, for example.

The body 10 also includes a charging port (not shown) at a bottom end 13 of the body 10.

The body 10 also includes a user interface device configured to convey information to a user. Here, the user interface device is implemented as a light 15, which may e.g. be configured to illuminate when the apparatus 1 is activated. Other user interface devices are possible, e.g. to convey information haptically or audibly to a user.

In this example, the consumable 30 has an opaque cap 31, a translucent tank 32 and a translucent window 33. When the consumable 30 is physically coupled to the body 10 as shown in Fig. 3A, only the cap 31 and window 33 can be seen, with the tank 32 being obscured from view by the body 10. The body 10 includes a slot 15 to accommodate the window 33. The window 33 is configured to allow the amount of liquid precursor 6 in the tank 32 to be visually assessed, even when the consumable 30 is physically coupled to the body 10.

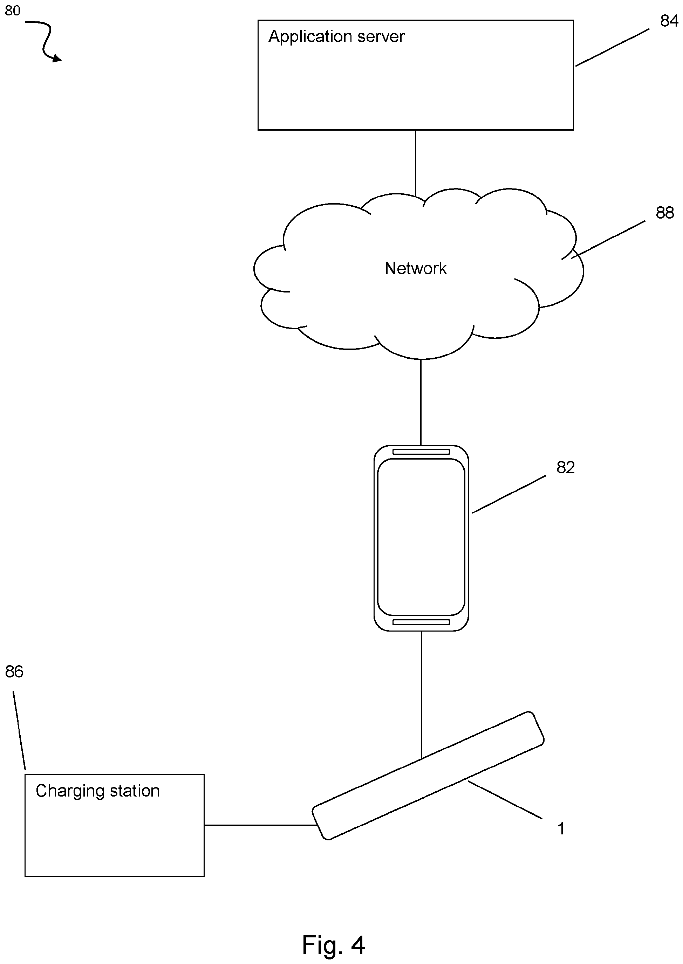

Fig. 4 shows an example system 80 for managing an aerosol generating apparatus 1, such as those described above with reference to any of Figs. 1-3B.

The system 80 as shown in Fig. 1 includes a mobile device 82, an application server 84, an optional charging station 86, as well as the aerosol generating apparatus 1.

In this example, aerosol generating apparatus 1 is configured to communicate wirelessly, e.g. via Bluetooth^{™}, with an application (or "app") installed on the mobile device 2, via a wireless interface included in the aerosol generating apparatus 1 and via a wireless interface included in the mobile device 82. The mobile device 82 may be a mobile phone, for example. The application on the mobile phone is configured to communicate with the application server 84, via a network 88. The application server 84 may utilise cloud storage, for example.

The network 88 may include a cellular network and/or the internet.

In other examples, the aerosol generating apparatus 1 may be configured to communicate with the application server 84 via a connection that does not involve the mobile device 82, e.g. via a narrowband internet of things ("NB-loT") or satellite connection. In some examples, the mobile device 82 may be omitted from the system 80.

A skilled person would readily appreciate that the mobile device 82 may be configured to communicate via the network 88 according to various communication channels, preferably a wireless communication channel such as via a cellular network (e.g. according to a standard protocol, such as 3G or 4G) or via a WiFi network.

The app installed on the mobile device 82 and the application server 84 may be configured to assist a user with managing their aerosol generating apparatus 1, based on information communicated between the aerosol generating apparatus 1 and the app, information communicated directly between the aerosol generating apparatus 1 and the application server 84, and/or information communicated between the app and the application server 84.

The charging station 86 (if present) may be configured to charge (and optionally communicate with) the aerosol generating apparatus 1, via a charging port on the aerosol generating apparatus 1. The charging port on the smoking substitute device 10 may be a USB port, for example, which may allow the aerosol generating apparatus 1 to be charged by any USB-compatible device capable of delivering power to the aerosol generating apparatus 1 via a suitable USB cable (in this case the USB-compatible device would be acting as the charging station 86). Alternatively, the charging station could be a docking station specifically configured to dock with the aerosol generating apparatus 1 and charge the aerosol generating apparatus 1 via the charging port on the aerosol generating apparatus 1.

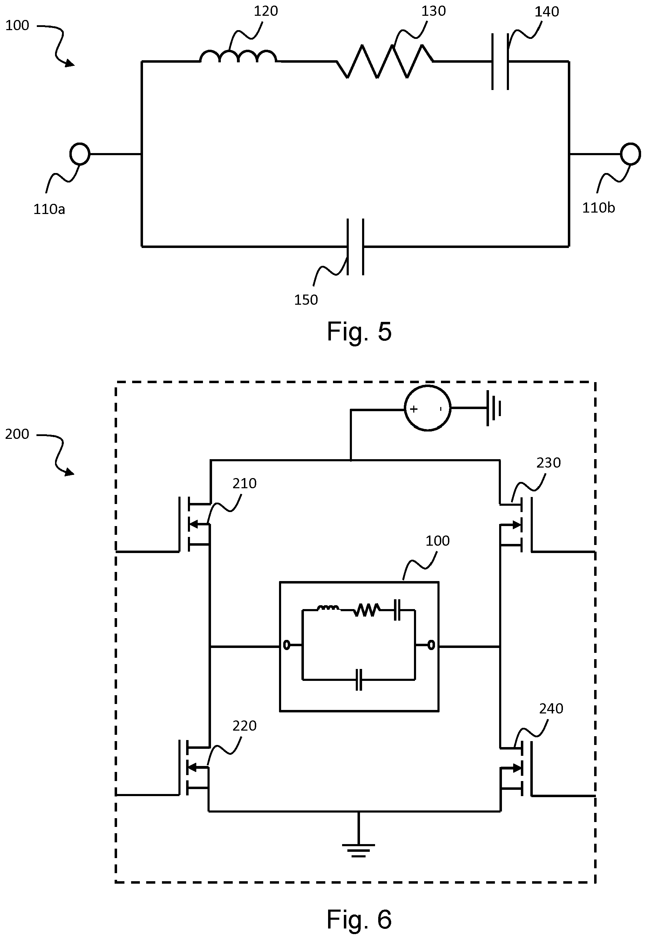

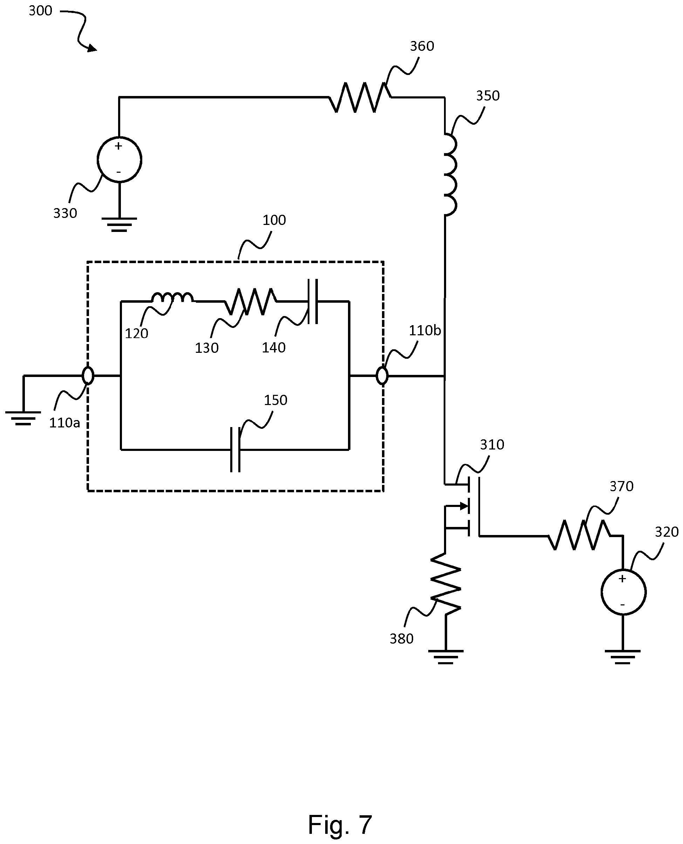

Fig. 5 shows an example of a circuit for modelling the behaviour of a piezoelectric transducer 100 at the resonant frequency of the piezoelectric transducer 100. Example circuits for providing a driving signal to the piezoelectric transducer 100 are described below with reference to Figures 6 and 7.

The circuit includes a set of components connected in series with each other between a pair of terminals 110a, 110b, including: an inductor 120; a resistor 130; and an in-series capacitor 140. The set of series components are connected in parallel with an in-parallel capacitor 150. Each of the components of the circuit model different aspects of the electrical and mechanical behaviour of the piezoelectric transducer 100.

The mechanical vibration of the piezoelectric transducer 100 is modelled by the inductive reactance of the inductor 120, when the frequency of the electric signal driving the piezoelectric transducer 100 is at, or near, the resonant frequency of the piezoelectric transducer 100.

Internal losses associated with the operation of the piezoelectric transducer 100, such as mechanical damping and dielectric losses within the piezoelectric crystal of the transducer 100 are modelled by the resistor 130. The resistance value of the resistor 130 is linked to the quality factor (or Q-factor) of the resonance of the piezoelectric transducer 100, which affects the amplitude and the sharpness of the resonance peak in the transducer's 100 frequency response.

Capacitive mechanical and electrical characteristics of the piezoelectric transducer 100 are modelled by the in-series capacitor 140.

Inherent dielectric properties of the material forming the piezoelectric transducer, e.g., due to the structure of the piezoelectric material between electrodes of the transducer 100 are modelled by the in-parallel capacitor 150. This inherent "parallel" capacitance significantly influences the resonance behaviour of the piezoelectric transducer 100, for example, by affecting the total impedance of the circuit at resonance when combined with the inductive and resistive elements (as modelled by the inductor 120 and the resistor 130).

As an example, the circuit of Figure 5 may be suitable for modelling a typical piezoelectric transducer 100 with a resonance frequency of approximately 3 MHz, by providing the inductor 120 with an inductance of 3 pH, the resistor 130 with a resistance of 4 Ω, the in-series capacitor 140 with a capacitance of 938 pF, and the in-parallel capacitor 150 with a capacitance of 1 nF.

Fig. 6 shows a portion of a conventional driving circuit 200 for driving a piezoelectric transducer 100 using an H-bridge. The H-bridge is defined by four switches 210, 220, 230, 240 arranged in an 'H-shaped' arrangement around the piezoelectric transducer 100. In the example shown in Fig. 6, the four switches 210, 220, 230, 240 are each defined by a respective MOSFET. The H-bridge of Fig. 6 is useful for rapidly changing the polarity of a voltage applied to the piezoelectric transducer 100, thereby driving piezoelectric vibrations in the transducer 100.

H-bridge circuits such as the one depicted in Fig. 6 may face challenges in the context of a user device such as the aerosol-generating apparatus 1 described herein.

For example, high-frequency switching of the four (MOSFET) switches 210, 220, 230, 240 may result in significant power and heat dissipation, generating considerable amounts of heat. This heat can degrade component performance over time and shorten the lifespan of the whole H-bridge, including the piezoelectric transducer 100. Moreover, the power dissipation may represent an undesirable inefficiency in the circuit performance of the H-bridge.

There may also be a risk of a latch-up type short-circuit in which one or more parts of the H-bridge circuit become uncontrollably conductive, thereby compromising the circuit's reliability. In the extreme, latch-up can lead to total circuit failure.

Electromagnetic interference may also be a concern when considering the implementation of a H-bridge. The rapid switching inherent in the operation of the four (MOSFET) switches 210, 220, 230, 240 can generate interference that can disrupt the operation of other electronic components/devices in the vicinity of the H-bridge.

Additionally, the operation of the piezoelectric transducer 100 (or indeed any component having an inductive load), can lead to high-voltage spikes in the current flowing through the circuit. Such spikes risk causing severe damage to the transistors used to embody the four MOSFET switches 210, 220, 230, 240 of the H-bridge shown in Fig. 6.

Furthermore, to induce high-frequency (e.g., ultrasonic) vibrations in the piezoelectric transducer 100, very precise and potentially complex timing control of the H-bridge is required. In particular, if both the first and second switches 210, 220, both the first and third switches 210, 230, both the second and fourth switches 220, 240 or both the third and fourth switches 230, 240 are open at the same time, there is a significant risk of shoot-through, or crossover, current that risks damaging the switches as the shoot-through current passes through and reduces the power efficiency of the H-bridge.

Fig. 7 shows an example of an improved driving circuit 300 for driving a piezoelectric transducer 100 using a single (MOSFET) switch 310.

The driving circuit 300 of Fig. 7 comprises a gate power source 320 configured to controllably apply a voltage to the gate of the MOSFET switch 310 to controllably open and close the MOSFET switch 310. The gate power source 320 may be connected to a clock, or may be an oscillator circuit so as to cyclically open and close the MOSFET switch 310 at a selected frequency.

The inventors have observed that the frequency of the opening and closing of the MOSFET switch 310, or the driving signal, is a parameter that directly affects the average size of droplets in the aerosol generated by the aerosol generating apparatuses described herein. In particular, when driving the piezoelectric transducer with a driving signal having a relatively higher driving frequency, a relatively smaller average droplet size is observed. This observation is consistent with the physical mechanism for cavitation described in Kooij et al. Sci. Rep., 9, 6128 (2019), the entirety of which is incorporated herein by reference.

The driving circuit 300 further comprises a driving power source 330 configured to supply power through the driving circuit 300. When the MOSFET switch 310 is closed, the current supplied by the driving power source 300 bypasses the piezoelectric transducer and flows into the source of the MOSFET switch 310 and out from the drain of the MOSFET switch 310 to ground. The driving power source 330 may be the power supply 2 discussed above in relation to Fig. 1.

When the MOSFET switch 310 is open, the current supplied by the driving power source 300 flows through the piezoelectric transducer 100 to ground, thereby inducing vibration in the piezoelectric transducer.

Application of a current to a piezoelectric transducer 100 induces a mechanical response in the transducer 100. Typically, the current applied to the piezoelectric transducer 100 is an alternating current so as to induce oscillatory vibrations in the piezoelectric transducer 100. Upon application of a current in a first polarity, opposite faces of the piezoelectric crystal of the transducer 100 respond by expanding, or bulging, outwards to define respective convex surfaces. Conversely, upon application of current in a second polarity opposite to the first polarity, the opposite faces of the piezoelectric crystal of the transducer 100 respond by contracting, or drawing, inwards to define respective concave surfaces. In the context of an aerosol-generating apparatus 1, it may be advantageous to only drive the piezoelectric transducer 100 in the first polarity so that physical contact between the transducer 100 and the at least some of the liquid precursor 6 can be maintained. Maintaining this physical contact improves the power efficiency of the inducement of cavitation in the liquid precursor 6, and therefore improves the efficiency of the generation of the aerosol. To this end, the driving signal provided by the driving power source 330 is preferably a direct current power source oscillating, at the piezoelectric transducer 100, between a maximum amplitude and a minimum (zero) amplitude with a frequency corresponding to the switching frequency of the MOSFET switch 310.

The driving circuit 300 may further comprise an inductor 350 connected in series with the piezoelectric transducer. The inductor 350 is arranged and configured with an inductance suitable for smoothing the current profile of the signal provided by the driving power source 330 such that the piezoelectric transducer 100 is not subjected to abrupt step-changes in the voltage and current flowing therethrough. This smoothing of the current profile consequently reduces the risk of damage to the piezoelectric transducer by reducing the risk of harmful voltage spikes.

The driving circuit 300 may further comprise one or more resistors 360, 370, 380 configured to limit the current flowing through the driving circuit.

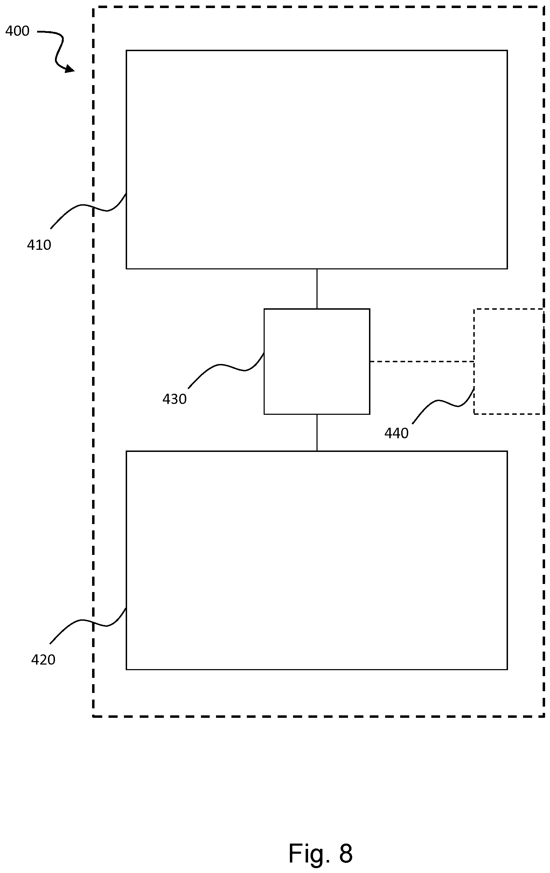

Referring to Fig. 8 there is provided an aerosol generating system 400 that comprises a first aerosol generator 410 for generating a first aerosol from a first formulation and a second aerosol generator 420 for generating a second aerosol from a second formulation, different from the first formulation.

Each of the first aerosol generator 410 and the second aerosol generator 420 comprises a piezoelectric transducer 100 and a drive circuit 300 as described above.

The first aerosol generator 410 is in fluid communication with a first tank containing a first liquid aerosol precursor, which may be a nicotine formulation, in order to communicate the first liquid aerosol precursor from the first tank to the first aerosol generator 410, and in particular to the aerosolization surface of the piezoelectric transducer of the first aerosol generator 410, for example by way of a wick.

The second aerosol generator 420 is in fluid communication with a second tank containing a second liquid aerosol precursor, which may be a flavour formulation, in order to communicate the second liquid aerosol precursor from the second tank to the second aerosol generator 420, and in particular to the aerosolization surface of the piezoelectric transducer of the second aerosol generator 420, for example by way of a wick.

Both the first aerosol generator 410 and the second aerosol generator 420 are communicatively linked to electrical circuitry 430 adapted to control the activation of the first aerosol generator 410 and the second aerosol generator 420 according to the methods described below.

Optionally, the aerosol generating system 400 comprises an inhalation sensor 440 communicatively linked to the electrical circuitry and adapted to sense an inhalation of the user. The electrical circuitry 430 may be adapted to activate the first aerosol generator 410 and the second aerosol generator 420 in response to an inhalation of the user sensed by the inhalation sensor 440. The inhalation sensor may be adapted to measure an inhalations strength of the user, for example as a pressure measurement.

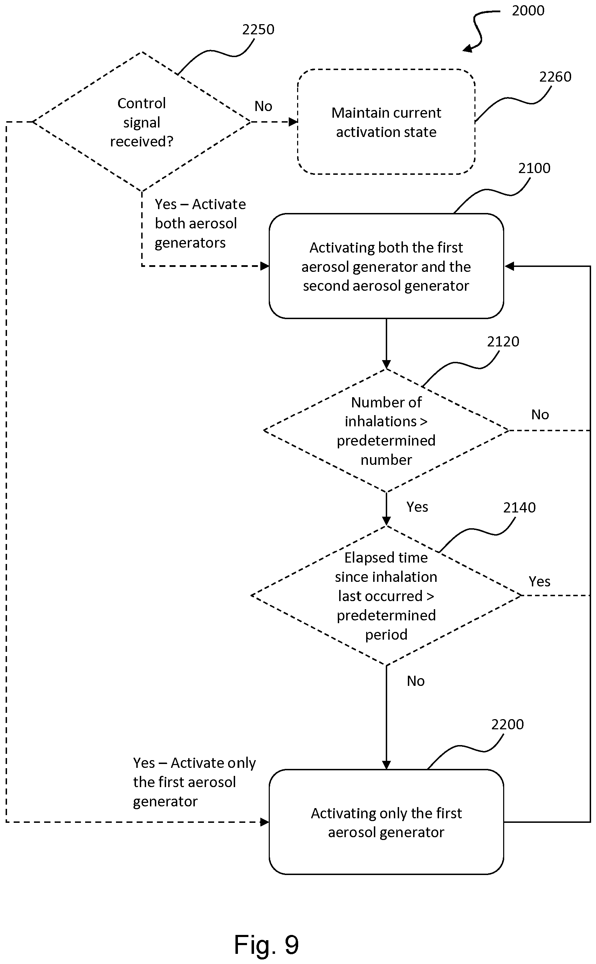

Referring to Fig. 9, there is illustrated a method 2000 for controlling an aerosol generating system 400 according to an aspect of the invention.

In the particular method shown in Fig. 9, the method 2000 is a method for alternating between activating both the first aerosol generator 410 and the second aerosol generator 420 and activating only the first aerosol generator 410 over a series of inhalations of a user of the aerosol generating system.

The method begins in step 2100 by activating both the first aerosol generator 410 and the second aerosol generator 420, for example, in response to an inhalation of the user. In response to a subsequent inhalation of the user, the method progresses to step 2200 by activating only the first aerosol generator 410.

After step 2200, the method returns to step 2100 and continues to alternate between activating both the first aerosol generator 410 and the second aerosol generator 420 and activating only the first aerosol generator 410 in response to the inhalations of the user.

Fig. 9 also shows several optional steps for controlling the aerosol generating system 400, and specifically, for controlling the alternation between activating both the first aerosol generator 410 and the second aerosol generator 420 and activating only the first aerosol generator 410.

The alternation between activating both the first aerosol generator 410 and the second aerosol generator 420 and activating only the first aerosol generator 410 may occur on consecutive inhalations. Put another way, the method may switch between steps 2100 and 2200 with each inhalation of the user.

In the example shown in Fig. 9, the method 2000 further comprises optional step 2120 in which it is checked whether the number of inhalations where both the first aerosol generator 410 and the second aerosol generator 420 are activated has exceed a predetermined number of inhalations.

If the number of inhalations where both the first aerosol generator 410 and the second aerosol generator 420 are activated has not exceed the predetermined number of inhalations, the method returns to step 2100 and both the first aerosol generator 410 and the second aerosol generator 420 are activated in response to the next inhalation of the user.

If the number of inhalations where both the first aerosol generator 410 and the second aerosol generator 420 are activated has exceed the predetermined number of inhalations, the method may then progress to step 2200 where only the first aerosol generator 410 is activated in response to the next inhalation of the user.

In the example shown in Fig. 9, there is no equivalent step to step 2120 during the return from step 2200 to step 2100. Accordingly, the step 2200 of activating only the first aerosol generator 410 only occurs for a single inhalation.

In the example shown in Fig. 9, the method 2000 further comprises optional step 2140 in which it is checked whether an elapsed time since an inhalation last occurred has exceeded a predetermined period.

If the elapsed time since an inhalation last occurred has exceeded the predetermined period, and one inhalation session (or series of inhalations) has stopped and a new inhalation session has effectively begun, the method returns to step 2100 and both the first aerosol generator 410 and the second aerosol generator 420 are activated in response to the next inhalation of the user.

If the elapsed time since an inhalation last occurred has not exceeded the predetermined period, the method may then progress to step 2200 where only the first aerosol generator 410 is activated in response to the next inhalation of the user.

In the example shown in Fig. 9, the method 2000 further comprises optional step 2250 in which it is checked whether a control signal has been received at the aerosol generating system 400, for example from mobile device 82. Step 2250 is outside of the alternating control loop as a control signal may be received at any time. The aerosol generating system 400 may perform step 2250 regularly, for example between each inhalation of the user or at a regular time interval.

If no control signal has been received at the aerosol generating system, the method progresses to step 2260 and the current activation state of the first aerosol generator 410 and the second aerosol generator 420, i.e., the activation states according to step 2100 or 2200, is maintained.

If a control signal has been received at the aerosol generating system, the method either progresses to step 2100 or step 2200 according to the nature of the control signal.

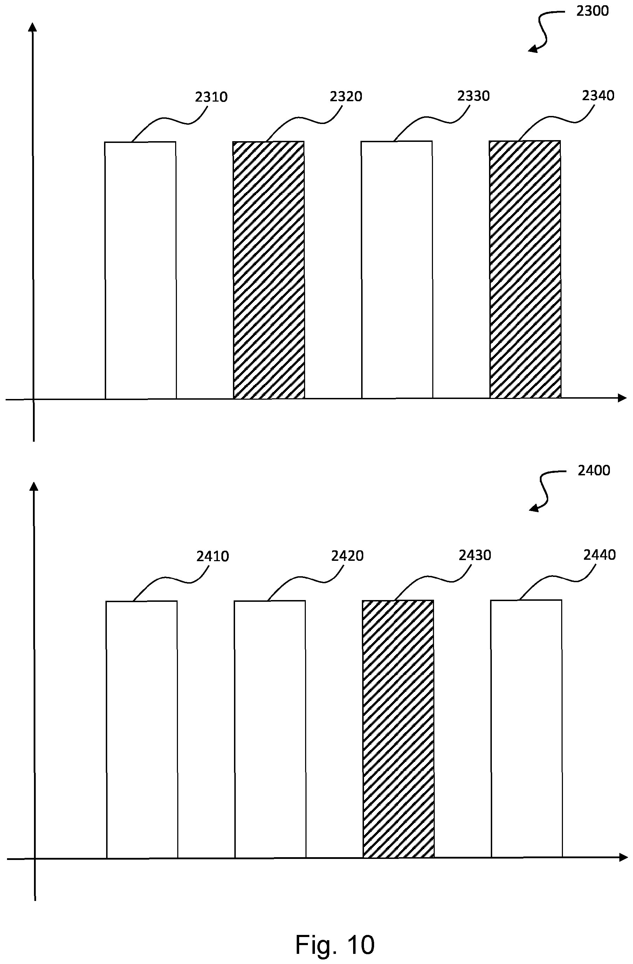

Fig. 10 shows a schematic representation of the driving scheme for the first aerosol generator 410 and the second aerosol generator according to the methods shown in Fig. 9.

Graphs 2300 and 2400 shows the activation states of the first aerosol generator 410 and the second aerosol generator 420 for four inhalations.

In graph 2300, the aerosol generating system 400 alternates between activating both the first aerosol generator 410 and the second aerosol generator 420, as represented by bars 2310 and 2330 which correspond to the first and third inhalations, and activating only the first aerosol generator 410, as represented by bars 2320 and 2340 which correspond to the second and fourth inhalations, on consecutive inhalations.

In graph 2400, the aerosol generating system 400 alternates between activating both the first aerosol generator 410 and the second aerosol generator 420, as represented by bars 2410, 2420 and 2440 which correspond to the first, second and fourth inhalations, and activating only the first aerosol generator 410, as represented by bar 2330 which corresponds to the third inhalation, on non-consecutive inhalations.

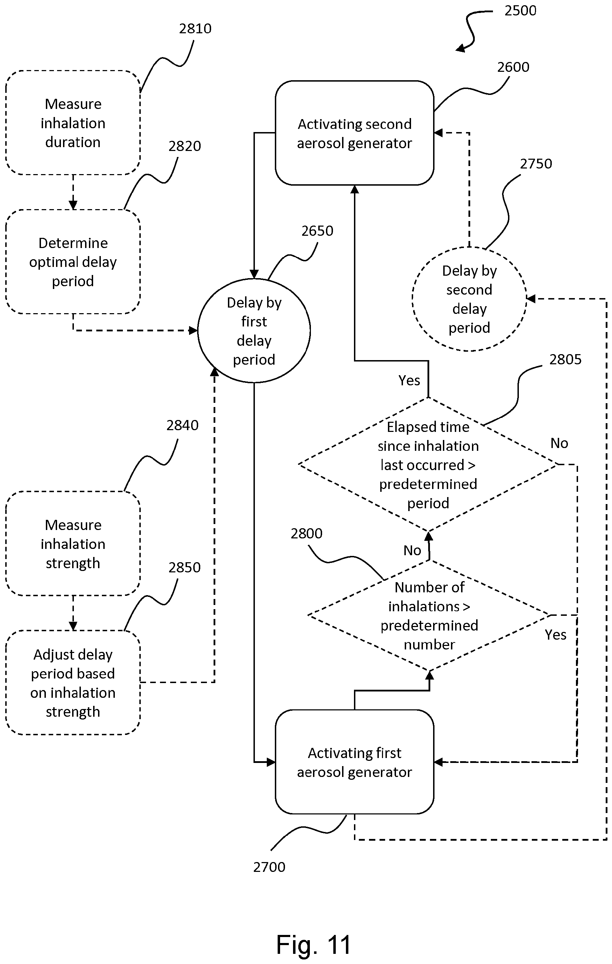

Referring to Fig. 11, there is illustrated a method 2500 for controlling an aerosol generating system 400 according to an aspect of the invention.

In the particular method shown in Fig. 11, the method 2500 is a method for delaying the activation of the first aerosol generator 410 with respect to the activation of the second aerosol generator 420 for a first delay period in response to an inhalation of a user.

The method begins in step 2600 by activating the second aerosol generator 420, for example, in response to an inhalation of the user. During the same inhalation, whilst the second aerosol generator 420 is generating aerosol, the method progresses to step 2650 and waits for a first delay period to elapse.

After the first delay period has elapsed in step 2650, and during the same inhalation, the method progresses to step 2700 by activating the first aerosol generator 410, such that both the first aerosol generator 410 and the second aerosol generator 420 are activated for the remainder of the inhalation.

After the end of the inhalation, the method returns to step 2600 ready for the next inhalation to occur at which point the method 2500 will repeat as outlined above, i.e., by activating the second aerosol generator and then activating the first aerosol generator after a first delay period.

Fig. 11 also shows several optional steps for controlling the aerosol generating system 400, and specifically, for controlling the timing of the activation of the first aerosol generator 410 and the second aerosol generator 420 relative to each other.

In some examples, the aerosol generating system 400 is adapted to alternate the delaying of the activation of the first aerosol generator 410 with respect to the activation of the second aerosol generator 420 for the first delay period with delaying the activation of the second aerosol generator 420 with respect to the activation of the first aerosol generator 410 for a second delay period.

Thus, in the example shown in Fig. 11, the method may alternate between beginning at step 2600 and step 2700.

The alternation between delaying of the activation of the first aerosol generator 410 with respect to the activation of the second aerosol generator 420 for the first delay period with delaying the activation of the second aerosol generator 420 with respect to the activation of the first aerosol generator 410 for a second delay period may occur on consecutive inhalations. Put another way, the method may switch between beginning at steps 2600 and 2700 with each inhalation of the user.

In the example shown in Fig. 11, the method 2500 further comprises optional step 2750 in which a second delay period is introduced between activating the first aerosol generator and activating the second aerosol generator.

In the example shown in Fig. 11, the method 2500 further comprises optional step 2800 in which it is checked whether the number of inhalations where the activation of the first aerosol generator 410 is delayed with respect to the activation of the second aerosol generator 420 (i.e., the number of inhalations where the method has progress from step 2600 to step 2650 and then to step 2700) has exceed a predetermined number of inhalations.

If the number of inhalations where the second aerosol generator 420 is activated before the first aerosol generator 410 has not exceed the predetermined number of inhalations, the method returns to step 2600 and second aerosol generator 420 continues to be activated before the first aerosol generator 410 for the next inhalation of the user.

If the number of inhalations where the second aerosol generator 420 is activated before the first aerosol generator 410 has exceed the predetermined number of inhalations, the method may then progress to step 2700 where the first aerosol generator 410 will be activated before the second aerosol generator 420 for the next inhalation of the user, for example by way of the second delay period in step 2750.

In the example shown in Fig. 11, there is no equivalent step to step 2800 during the return from step 2600 to step 2700. Accordingly, the method running from step 2700 to 2600, via step 2750, (i.e., the delaying of the activation of the second aerosol generator with respect to the first aerosol generator) only occurs for a single inhalation.

In the example shown in Fig. 11, the method 2500 further comprises optional step 2805 in which it is checked whether an elapsed time since an inhalation last occurred has exceeded a predetermined period.

If the elapsed time since an inhalation last occurred has exceeded the predetermined period, and one inhalation session (or series of inhalations) has stopped and a new inhalation session has effectively begun, the method returns to step 2600 and the activation of the first aerosol generator is delayed with respect to the activation of the second aerosol generator in response to the next inhalation of the user.

If the elapsed time since an inhalation last occurred has not exceeded the predetermined period, the method may then progress to step 2700 where the first aerosol generator 410 will be activated before the second aerosol generator 420 for the next inhalation of the user, for example by way of the second delay period in step 2750.

In the example shown in Fig. 11, the method 2500 further comprises the optional step 2810 of measuring the inhalation duration of the user over a series of inhalations. In step 2820, an optimal delay period, which may be the first delay period in step 2650 or the second delay period in step 2750, for delaying the activation of the first/second aerosol generator with respect to the activation of the second/first aerosol generator based on the average inhalation duration of the user.

In the example shown in Fig. 11, the method 2500 further comprises optional step 2840 of measuring the inhalation strength of the user over a series of inhalations, for example by way of inhalation sensor 440. In step 2850, the delay period, which may be the first delay period in step 2650 or the second delay period in step 2750, is adjusted based on the average inhalation strength of the user.

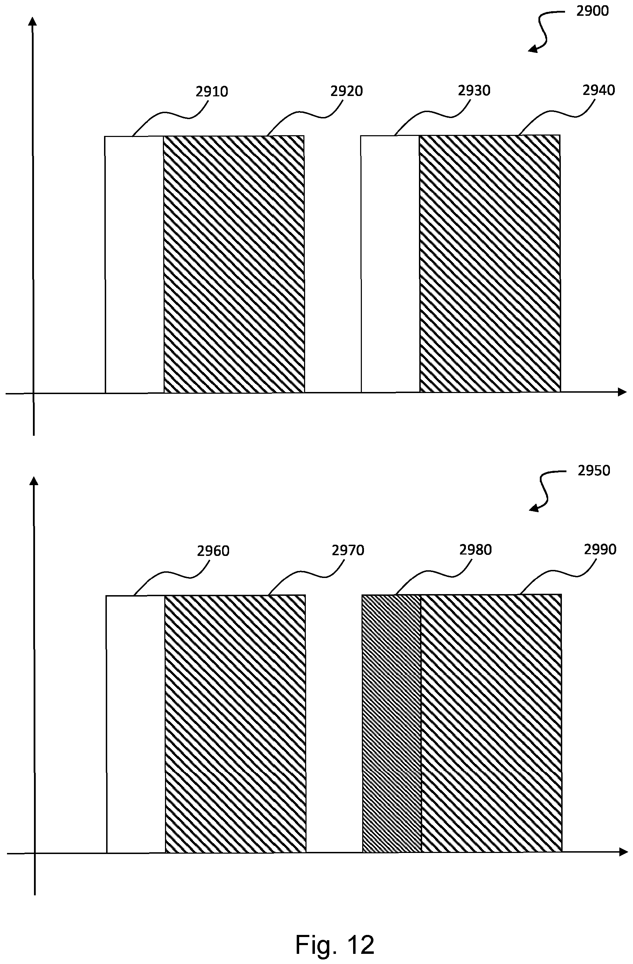

Fig. 12 shows a schematic representation of the driving scheme for two aerosol generating units according to the method shown in Fig. 11.

Graphs 2900 and 2950 shows the activation states of the first aerosol generator 410 and the second aerosol generator 420 for two inhalations.

In graph 2900, the aerosol generating system 400 delays the activation of the first aerosol generator 410 with respect to the activation of the second aerosol generator 420, such that for the first portion of the inhalations only the second aerosol generator 420 is active, as represented by bars 2910 and 2930, and for the second portion of the inhalations both the first aerosol generator 410 and the second aerosol generator 420 are active, as represented by bars 2920 and 2940.

In graph 2950, the aerosol generating system 400 alternates between delaying the activation of the first aerosol generator 410 with respect to the activation of the second aerosol generator 420 in the first inhalation and delaying the activation of the second aerosol generator 420 with respect to the activation of the first aerosol generator 410 in the second inhalation.

In the first inhalation shown in graph 2950, the aerosol generating system 400 delays the activation of the first aerosol generator 410 with respect to the activation of the second aerosol generator 420, such that for the first portion of the inhalation only the second aerosol generator 420 is active, as represented by bar 2960, and for the second portion of the first inhalation both the first aerosol generator 410 and the second aerosol generator 420 are active, as represented by bar 2970.

In the second inhalation shown in graph 2950, the aerosol generating system 400 delays the activation of the second aerosol generator 420 with respect to the activation of the first aerosol generator 410, such that for the first portion of the inhalation only the first aerosol generator 410 is active, as represented by bar 2980, and for the second portion of the second inhalation both the first aerosol generator 410 and the second aerosol generator 420 are active, as represented by bar 2990.

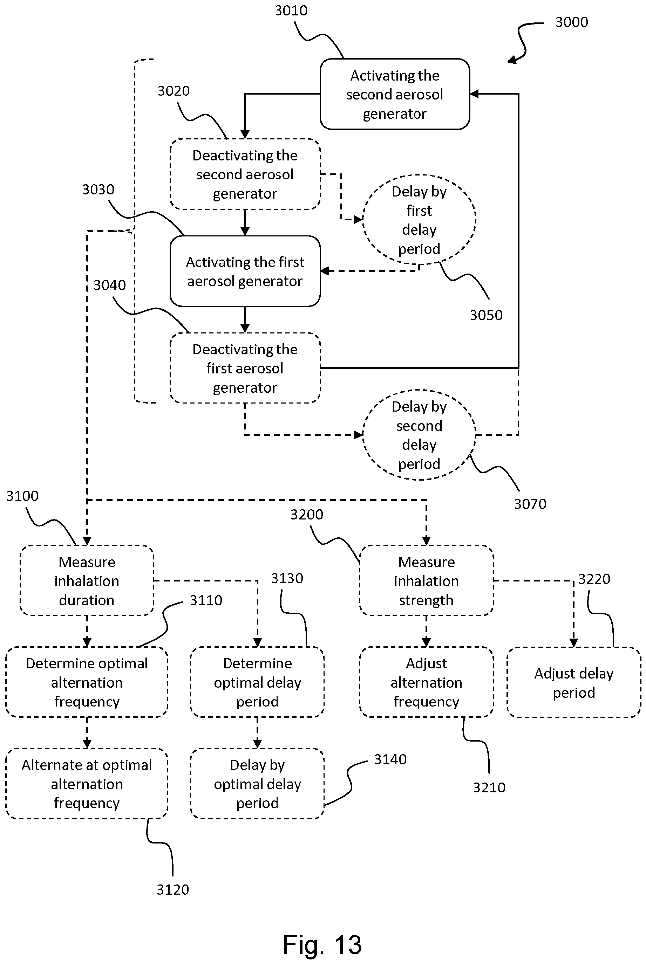

Referring to Fig. 13, there is illustrated a method 3000 for controlling an aerosol generating system 400 according to an aspect of the invention.

In the particular method shown in Fig. 13, the method 3000 is a method for modulating the activation of the first aerosol generator 410 and the second aerosol generator 420 over a single inhalation period of a user of the aerosol generating system. Fig. 13 illustrates two different methods of modulating the activation of the first aerosol generator 410 and the second aerosol generator 420 over a single inhalation period, which are alternating an activation state of the first and second aerosol generators over the single inhalation period and delaying the activation state of one of the first and second aerosol generators with respect to another of the first and second aerosol generators over the single inhalation period.

In the example where modulating the activation of the first aerosol generator 410 and the second aerosol generator 420 comprises alternating an activation state of the first and second aerosol generators over the single inhalation period, the method begins in step 3010 by activating the second aerosol generator 420, for example, in response to an inhalation of the user.

The method may then progress to optional step 3020, where the second aerosol generator 420 is deactivated. Within the same inhalation period, the method progresses to step 3030, where the first aerosol generator 410 is activated. If the second aerosol generator 420 is deactivated in step 3020, then only the first aerosol generator 420 will be activated at step 3030. If the second aerosol generator 420 is not deactivated in step 3020, or step 3020 is not present, then both the first aerosol generator 410 and the second aerosol generator 420 will be activate at step 3030.

In the example where modulating the activation of the first aerosol generator 410 and the second aerosol generator 420 further comprises delaying the activation state of one of the first aerosol generator 410 and second aerosol generator 420 with respect to the other of the first aerosol generator 410 and the second aerosol generator 420, the method may progress to optional step 3050 and wait for a first delay period to elapse.

After the first delay period has elapsed in step 3050, and during the same inhalation, the method progresses to step 3030 by activating the first aerosol generator 410, such that for a period of time within the single inhalation period, neither aerosol generator is active between the activation of the second aerosol generator 420 and the first aerosol generator 410.

The method may then progress to optional step 3040, where the first aerosol generator 410 is deactivated. Still within the same inhalation period, the method may return to step 3010, where the second aerosol generator 420 is activated. If the first aerosol generator 410 is deactivated in step 3040, then only the second aerosol generator 420 will be activated at step 3010. If the first aerosol generator 410 is not deactivated in step 3040, or step 3040 is not present, then both the first aerosol generator 410 and the second aerosol generator 420 will be activate at step 3010.

In the example where modulating the activation of the first aerosol generator 410 and the second aerosol generator 420 further comprises delaying the activation state of one of the first aerosol generator 410 and second aerosol generator 420 with respect to the other of the first aerosol generator 410 and the second aerosol generator 420, the method may progress to optional step 3050 and wait for a second delay period to elapse.

After the second delay period has elapsed in step 3070, and during the same inhalation, the method returns to step 3010 by activating the second aerosol generator 420, such that for a period of time within the single inhalation period, neither aerosol generator is active between the activation of the first aerosol generator 410 and the second aerosol generator 420.

Thus, over a single inhalation period, the aerosol generating system 400 may: alternate the activation of only the first aerosol generator 410 and only the second aerosol generator 420; alternate the activation of only the first aerosol generator 410 and both the first aerosol generator 410 and the second aerosol generator 420; and alternate the activation of only the second aerosol generator 420 and both the first aerosol generator 410 and the second aerosol generator 420.

Fig. 13 also shows several optional steps for controlling the aerosol generating system 400, and specifically, for modulating the activation of the first aerosol generator 410 and the second aerosol generator 420.

In the example shown in Fig. 13, the method 3000 further comprises the optional step 3100 of measuring the inhalation duration of the user over a series of inhalations.

The method may progress to step 3110 where an optimal alternation frequency for alternating between activation states of the first and second aerosol generators is determined based on an average inhalation duration of the user. In step 3120, the aerosol generating system uses the optimal alternation frequency to alternate the activation state of the first and second aerosol generators.

In the example where modulating the activation of the first aerosol generator 410 and the second aerosol generator 420 further comprises delaying the activation state of one of the first aerosol generator 410 and second aerosol generator 420 with respect to the other of the first aerosol generator 410 and the second aerosol generator 420, the method may progress to step 3130 where an optimal delay period is determined based on the average inhalation duration of the user. The optimal delay period may be the first delay period in step 3050 or the second delay period in step 3070, for delaying the activation of the first/second aerosol generator with respect to the activation of the second/first aerosol generator. In step 3140, the aerosol generating system uses the optimal delay period to delay the activation of the first/second aerosol generator with respect to the activation of the second/first aerosol generator.

In the example shown in Fig. 13, the method 3000 further comprises optional step 3200 of measuring the inhalation strength of the user over a series of inhalations, for example by way of inhalation sensor 440.

The method may progress to step 3210 where the aerosol generating system adjusts the alternation frequency for alternating the activation states of the first and second aerosol generators based on the average inhalation strength.

In the example where modulating the activation of the first aerosol generator 410 and the second aerosol generator 420 further comprises delaying the activation state of one of the first aerosol generator 410 and second aerosol generator 420 with respect to the other of the first aerosol generator 410 and the second aerosol generator 420, the method may progress to step 3220 where the aerosol generating system adjusts the first or second delay period based on the average inhalation strength.

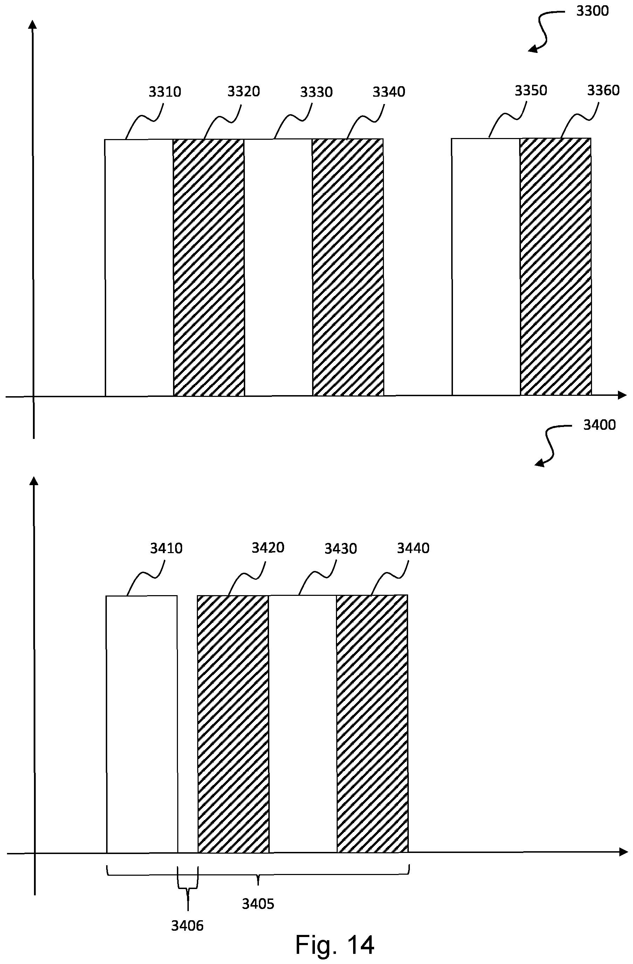

Fig. 14 shows a schematic representation of the driving scheme for the first aerosol generator 410 and the second aerosol generator 420 according to the methods shown in Fig. 13.

Graphs 3300 and 3400 shows the activation states of the first aerosol generator 410 and the second aerosol generator 420 for two inhalations.

In graph 3300, the aerosol generating system 400 alternates between activating the second aerosol generator 420, as represented by bars 3310, 3330 and 3350, which correspond to portions of the inhalations, and activating only the first aerosol generator 410, as represented by bars 3320, 3340 and 3360, which correspond to the remaining portions of the inhalations.

In graph 3400, the aerosol generating system 400 delays the activation of the first aerosol generator 410 with respect to the activation of the second aerosol generator 420 between the first activation of the second aerosol generator 420 and the first activation of the first aerosol generator 410.

For the first portion of the inhalation 3405 only the second aerosol generator 420 is active, as represented by bar 3410, followed by a period 3406, i.e., the first delay period, where neither of the first aerosol generator 410 and the second aerosol generator 420 are active. For the remainder of the inhalation 3405, the aerosol generating system 400 alternates between activating the second aerosol generator 420, as represented by bar 3430, and activating only the first aerosol generator 410, as represented by bars 3420 and 3440.

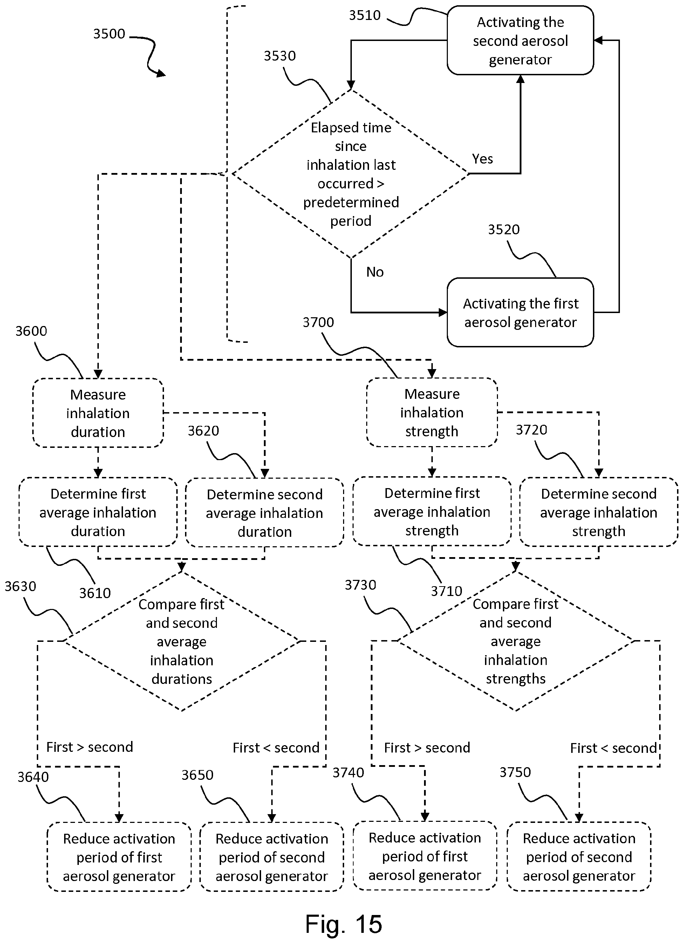

Referring to Fig. 15, there is illustrated a method 3500 for controlling an aerosol generating system 400 according to an aspect of the invention.

In the particular method shown in Fig. 15, the method 3500 is a method for alternating between activating only the second aerosol generator 420 and activating only the first aerosol generator 410 over on consecutive of inhalations of a user of the aerosol generating system.

The method begins in step 3510 by activating the second aerosol generator 420, for example, in response to an inhalation of the user. In response to a subsequent inhalation of the user, the method progresses to step 3520 by activating only the first aerosol generator 410.

After step 3520, the method returns to step 3510 and continues to alternate between activating only the second aerosol generator 420 and activating only the first aerosol generator 410 with each inhalation of the user.

Fig. 15 also shows several optional steps for controlling the aerosol generating system 400, and specifically, for controlling the alternation between activating the second aerosol generator 420 and activating the first aerosol generator 410.

The alternation between activating only the second aerosol generator 420 and activating only the first aerosol generator 410 occurs on consecutive inhalations. Put another way, the method switches between steps 3510 and 3520 with each inhalation of the user.

In the example shown in Fig. 15, the method 3500 further comprises optional step 3530 in which it is checked whether an elapsed time since an inhalation last occurred has exceeded a predetermined period.

If the elapsed time since an inhalation last occurred has exceeded the predetermined period, and one inhalation session (or series of inhalations) has stopped and a new inhalation session has effectively begun, the method returns to step 3510 and the second aerosol generator 420 is activated in response to the next inhalation of the user.

If the elapsed time since an inhalation last occurred has not exceeded the predetermined period, the method may then progress to step 3520 where the first aerosol generator 410 is activated in response to the next inhalation of the user.

In the example shown in Fig. 15, the method 3500 further comprises the optional step 3600 of measuring the inhalation duration of the user over a series of inhalations.

In step 3610, the first average inhalation duration for inhalations when the first aerosol generator 410 is active is determined. In step 3620, the second average inhalation duration for inhalations when the second aerosol generator 420 is active is determined.

In step 3630, the first and second average inhalation durations are compared. If the first average inhalation duration is greater than the second average inhalation duration, the method progresses to step 3640 and the first activation period of the first aerosol generator 410 is reduced. The first activation period is a portion of the first average inhalation time. If the second average inhalation duration is greater than the first average inhalation duration, the method progresses to step 3650 and the second activation period of the second aerosol generator is reduced. The second activation period is a portion of the second average inhalation time.

In the example shown in Fig. 15, the method 3500 further comprises optional step 3700 of measuring the inhalation strength of the user over a series of inhalations.

In step 3610, the first average inhalation strength for inhalations when the first aerosol generator 410 is active is determined. In step 3620, the second average inhalation strength for inhalations when the second aerosol generator 420 is active is determined.

In step 3630, the first and second average inhalation strengths are compared. If the first average inhalation strength is greater than the second average inhalation strength, the method progresses to step 3640 and the first activation period of the first aerosol generator 410 is reduced. The first activation period is a portion of the first average inhalation time. If the second average inhalation strength is greater than the first average inhalation strength, the method progresses to step 3650 and the second activation period of the second aerosol generator is reduced. The second activation period is a portion of the second average inhalation time.

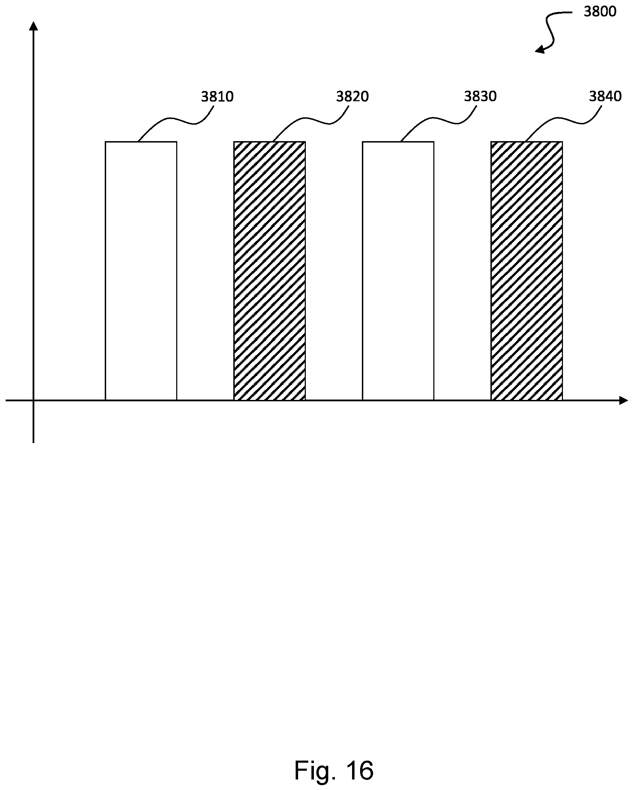

Fig. 16 shows a schematic representation of the driving scheme for the first aerosol generator 410 and the second aerosol generator 420 according to the methods shown in Fig. 15.

Graph 3800 shows the activation states of the first aerosol generator 410 and the second aerosol generator 420 for four inhalations.

In graph 3800, the aerosol generating system 400 alternates between activating only the second aerosol generator 420, as represented by bars 3810 and 3830 which correspond to the first and third inhalations, and activating only the first aerosol generator 410, as represented by bars 3820 and 3840 which correspond to the second and fourth inhalations, on consecutive inhalations.

### REFERENCES

The entirety of the following documents, which are referenced in the present disclosure, are incorporated by reference into the present disclosure:
- Kooij, S., Astefanei, A., Corthals, G.L. et al. Size distributions of droplets produced by ultrasonic nebulizers. Sci Rep 9, 6128 (2019). https://doi.org/10.1038/s41598-019-42599-8

## Claims

1. An aerosol generating system comprising:
a first aerosol generator for generating a first aerosol from a first formulation, wherein the first formulation comprises a nicotine formulation, the first aerosol being for inhalation by a user;
a second aerosol generator for generating a second aerosol from a second formulation, different from the first formulation, wherein the second formulation comprises a flavour formulation the second aerosol being for inhalation by a user in combination with the first aerosol; and
electrical circuitry, wherein the electrical circuitry is adapted to:
delay the activation of the first aerosol generator with respect to the activation of the second aerosol generator for a first delay period in response to an inhalation of a user of the aerosol generating system.

2. The aerosol generating system claimed in claim 1, wherein the electrical circuitry is further adapted to:
measure an inhalation duration of the user over a series of inhalations;
determine an optimal first delay period for delaying the activation of the first aerosol generator with respect to the activation of the second aerosol generator based on an average inhalation duration of the user; and
delaying the activation of the first aerosol generator with respect to the activation of the second aerosol generator for the optimal first delay period.

3. The aerosol generating system claimed in claims 1 or 2, wherein the electrical circuitry is further adapted to alternate the delaying of the activation of the first aerosol generator with respect to the activation of the second aerosol generator for the first delay period with delaying the activation of the second aerosol generator with respect to the activation of the first aerosol generator for a second delay period.

4. The aerosol generating system claimed in claim 3, wherein the alternating between the delaying of the activation of the first aerosol generator with respect to the activation of the second aerosol generator and delaying the activation of the second aerosol generator with respect to the activation of the first aerosol generator is performed on non-consecutive inhalations of the user of the aerosol generating system.

5. The aerosol generating system claimed in claim 4, wherein alternating between the delaying of the activation of the first aerosol generator with respect to the activation of the second aerosol generator and delaying the activation of the second aerosol generator with respect to the activation of the first aerosol generator comprises:
delaying the activation of the first aerosol generator with respect to the activation of the second aerosol generator for one or more inhalations of the user of the aerosol generating system before switching to delaying the activation of the second aerosol generator with respect to the activation of the first aerosol generator; and
delaying the activation of the second aerosol generator with respect to the activation of the first aerosol generator for a single inhalation of the user of the aerosol generating system before switching to delaying the activation of the first aerosol generator with respect to the activation of the second aerosol generator.

6. The aerosol generating system claimed in any preceding claim, wherein the electrical circuitry is further adapted to:
determine an elapsed time since an inhalation last occurred; and
if the elapsed time exceeds a predetermined period, switching to delaying the activation of the first aerosol generator with respect to the activation of the second aerosol generator; and
if the elapsed time exceeds the predetermined period, maintaining a current delayed activation state of the first and second aerosol generators.

7. The aerosol generating system claimed in any preceding claim, wherein the aerosol generating system further comprises a pressure sensor adapted to sense an inhalation of the user and send an inhalation signal to the electrical circuitry in response to sensing the inhalation of the user, and wherein the electrical circuitry is adapted to activate the first and second aerosol generators in response to the inhalation signal.

8. The aerosol generating system claimed in claim 7, wherein the electrical circuitry is further adapted to:
determine an inhalation strength based on the inhalation signal; and
adjust the first delay period based on the inhalation strength.

9. The aerosol generating system claimed in any preceding claim, wherein the aerosol generating system further comprises a communication unit adapted to receive a control signal, and wherein the electrical circuitry is adapted to adjust a function of the aerosol generating system in response to the control signal.

10. The aerosol generating system claimed in claim 9, wherein adjusting a function of the aerosol generating system comprises adjusting the first delay period between activating the second aerosol generator and activating the first aerosol generator.

11. The aerosol generating system claimed in any preceding claim, wherein the first aerosol generator comprises a first piezoelectric transducer and the second aerosol generator comprises a second piezoelectric transducer.

12. A computer-implemented method for controlling an aerosol generating system, the aerosol generating system comprising:
a first aerosol generator for generating a first aerosol from a first formulation; and
a second aerosol generator for generating a second aerosol from a second formulation, different from the first formulation,
and wherein the computer-implemented method comprises:
delaying the activation of the first aerosol generator with respect to the activation of the second aerosol generator for a first delay period in response to an inhalation of a user of the aerosol generating system.

13. The computer implemented method of claim 12, wherein the computer implemented method further comprises:
measuring an inhalation duration of the user over a series of inhalations;
determining an optimal first delay period for delaying the activation of the first aerosol generator with respect to the activation of the second aerosol generator based on an average inhalation duration of the user; and
delaying the activation of the first aerosol generator with respect to the activation of the second aerosol generator for the optimal first delay period.

14. A computer-readable storage medium comprising instructions which, when executed by electrical circuitry of an aerosol generating system, cause the electrical circuitry to carry out the method of claims 12 to 13.

15. Electrical circuitry for an aerosol generating system, the electrical circuitry being arranged to perform the methods of claims 12 to 13.
